# EUROPEAN PATENT SPECIFICATION

(11) **EP 2 767 592 B1**
(45) Date of publication and mention of the grant of the patent: **28.06.2017**
(21) Application number: 12839616.5
(22) Date of filing: 11.10.2012
(51) Int. Cl.: C12Q 1/68, C12Q 1/02, C12N 15/09, G01N 33/50

(54) **MUTAGENICITY TEST METHOD USING MAMMALIAN CELLS**
MUTAGENITÄTSTESTVERFAHREN UNTER VERWENDUNG VON SÄUGETIERZELLEN
PROCÉDÉ DE TEST DE MUTAGÉNICITÉ UTILISANT DES CELLULES MAMMALIENNES

(30) Priority: 11.10.2011 JP 2011224292
(43) Date of publication of application: 20.08.2014
(73) Proprietor: Nissin Foods Holdings Co., Ltd., Osaka-shi, Osaka 532-8524 (JP)
(72) Inventor: MIZOTA, Taisei, Osaka-shi Osaka 532-8524 (JP); OHNO, Katsutoshi, Osaka-shi Osaka 532-8524 (JP); YAMADA, Toshihiro, Osaka-shi Osaka 532-8524 (JP)
(74) Representative: Müller-Boré & Partner Patentanwälte PartG mbB
(86) International application number: PCT/JP2012/076325
(87) International publication number: WO 2013/054846

(56) References cited:
- WO-A1-2008/105476
- WO-A1-2009/028418
- JP-A- 2005 000 024
- OHNO K ET AL: "A genotoxicity test system based on p53R2 gene expression in human cells: Assessment of its reactivity to various classes of genotoxic chemicals", MUTATION RESEARCH. GENETIC TOXICOLOGY AND ENVIRONMENTAL MUTAGENESIS, ELSEVIER, AMSTERDAM, NL, vol. 656, no. 1-2, 30 October 2008 (2008-10-30), pages 27-35, XP025507086, ISSN: 1383-5718, DOI: 10.1016/J.MRGENTOX.2008.07.002 [retrieved on 2008-07-16]
- TAISEI MIZOTA ET AL: "Validation of a genotoxicity test based on2 gene expression in human lymphoblastoid cells", MUTATION RESEARCH. GENETIC TOXICOLOGY AND ENVIRONMENTAL MUTAGENESIS, ELSEVIER, AMSTERDAM, NL, vol. 724, no. 1, 12 June 2011 (2011-06-12) , pages 76-85, XP028252525, ISSN: 1383-5718, DOI: 10.1016/J.MRGENTOX.2011.06.003 [retrieved on 2011-06-17]
- MIZOTA T. ET AL.: 'Validation of a genotoxicity test based on p53R2 gene expression in human lymphoblastoid cells' MUTAT RES. vol. 724, 17 June 2011, pages 76 - 85, XP028252525
- OHNO K. ET AL.: 'Genotoxicity test system based on p53R2 gene expression in human cells: examination with 80 chemicals' MUTAT RES. vol. 588, 2005, pages 47 - 57, XP027658927
- OHNO K. ET AL.: 'A genotoxicity test system based on p53R2 gene expression in human cells: assessment of its reactivity to various classes of genotoxic chemicals' MUTAT RES. vol. 656, 2008, pages 27 - 35, XP025507086

### SEQUENCE LISTING

<110> NISSIN FOODS HOLDINGS CO., LTD.
<120> Genotoxicity test using mammalian cells
<130> P12-135
<150> JP 2011-224292
   <151> 2011-10-11
<160> 20
<170> PatentIn version 3.4
<210> 1
   <211> 10
   <212> DNA
   <213> mammalian
<400> 1
   wrrcwwgyyy 10
<210> 2
   <211> 20
   <212> DNA
   <213> mammalian
<400> 2
   wrrcwwgyyy wrrcwwgyyy 20
<210> 3
   <211> 20
   <212> DNA
   <213> mammalian
<400> 3
   tgacatgccc aggcatgtct 20
<210> 4
   <211> 20
   <212> DNA
   <213> mammalian
<400> 4
   gggcaggccc gggcttgtcg 20
<210> 5
   <211> 20
   <212> DNA
   <213> mammalian
<400> 5
   tctcttgccc gggcttgtcg 20
<210> 6
   <211> 20
   <212> DNA
   <213> mammalian
<400> 6
   gaacttgggg gaacatgttt 20
<210> 7
   <211> 20
   <212> DNA
   <213> mammalian
<400> 7
   gaacatgtcc caacatgttg 20
<210> 8
   <211> 1653
   <212> DNA
   <213> Artificial
<220>
   <223> luc2 gene
<400> 8
   atggaagatg ccaaaaacat taagaagggc ccagcgccat tctacccact cgaagacggg     60
   accgccggcg agcagctgca caaagccatg aagcgctacg ccctggtgcc cggcaccatc    120
   gcctttaccg acgcacatat cgaggtggac attacctacg ccgagtactt cgagatgagc    180
   gttcggctgg cagaagctat gaagcgctat gggctgaata caaaccatcg gatcgtggtg    240
   tgcagcgaga atagcttgca gttcttcatg cccgtgttgg gtgccctgtt catcggtgtg    300
   gctgtggccc cagctaacga catctacaac gagcgcgagc tgctgaacag catgggcatc    360
   agccagccca ccgtcgtatt cgtgagcaag aaagggctgc aaaagatcct caacgtgcaa    420
   aagaagctac cgatcataca aaagatcatc atcatggata gcaagaccga ctaccagggc    480
   ttccaaagca tgtacacctt cgtgacttcc catttgccac ccggcttcaa cgagtacgac    540
   ttcgtgcccg agagcttcga ccgggacaaa accatcgccc tgatcatgaa cagtagtggc    600
   agtaccggat tgcccaaggg cgtagcccta ccgcaccgca ccgcttgtgt ccgattcagt    660
   catgcccgcg accccatctt cggcaaccag atcatccccg acaccgctat cctcagcgtg    720
   gtgccatttc accacggctt cggcatgttc accacgctgg gctacttgat ctgcggcttt    780
   cgggtcgtgc tcatgtaccg cttcgaggag gagctattct tgcgcagctt gcaagactat    840
   aagattcaat ctgccctgct ggtgcccaca ctatttagct tcttcgctaa gagcactctc    900
   atcgacaagt acgacctaag caacttgcac gagatcgcca gcggcggggc gccgctcagc    960
   aaggaggtag gtgaggccgt ggccaaacgc ttccacctac caggcatccg ccagggctac   1020
   ggcctgacag aaacaaccag cgccattctg atcacccccg aaggggacga caagcctggc   1080
   gcagtaggca aggtggtgcc cttcttcgag gctaaggtgg tggacttgga caccggtaag   1140
   acactgggtg tgaaccagcg cggcgagctg tgcgtccgtg gccccatgat catgagcggc   1200
   tacgttaaca accccgaggc tacaaacgct ctcatcgaca aggacggctg gctgcacagc   1260
   ggcgacatcg cctactggga cgaggacgag cacttcttca tcgtggaccg gctgaagagc   1320
   ctgatcaaat acaagggcta ccaggtagcc ccagccgaac tggagagcat cctgctgcaa   1380
   caccccaaca tcttcgacgc cggggtcgcc ggcctgcccg acgacgatgc cggcgagctg   1440
   cccgccgcag tcgtcgtgct ggaacacggt aaaaccatga ccgagaagga gatcgtggac   1500
   tatgtggcca gccaggttac aaccgccaag aagctgcgcg gtggtgttgt gttcgtggac   1560
   gaggtgccta aaggactgac cggcaagttg gacgcccgca agatccgcga gattctcatt   1620
   aaggccaaga agggcggcaa gatcgccgtg taa                                1653
<210> 9
   <211> 4242
   <212> DNA
   <213> Artificial
<220>
   <223> plasmid
<400> 9
   ggcctaactg gccggtacct gagctcgcta gcctcgagga tatcaagatc tggcctcggc     60
   ggccaagctt ggcaatccgg tactgttggt aaagccacca tggaagatgc caaaaacatt    120
   aagaagggcc cagcgccatt ctacccactc gaagacggga ccgccggcga gcagctgcac    180
   aaagccatga agcgctacgc cctggtgccc ggcaccatcg cctttaccga cgcacatatc    240
   gaggtggaca ttacctacgc cgagtacttc gagatgagcg ttcggctggc agaagctatg    300
   aagcgctatg ggctgaatac aaaccatcgg atcgtggtgt gcagcgagaa tagcttgcag    360
   ttcttcatgc ccgtgttggg tgccctgttc atcggtgtgg ctgtggcccc agctaacgac    420
   atctacaacg agcgcgagct gctgaacagc atgggcatca gccagcccac cgtcgtattc    480
   gtgagcaaga aagggctgca aaagatcctc aacgtgcaaa agaagctacc gatcatacaa    540
   aagatcatca tcatggatag caagaccgac taccagggct tccaaagcat gtacaccttc    600
   gtgacttccc atttgccacc cggcttcaac gagtacgact tcgtgcccga gagcttcgac    660
   cgggacaaaa ccatcgccct gatcatgaac agtagtggca gtaccggatt gcccaagggc    720
   gtagccctac cgcaccgcac cgcttgtgtc cgattcagtc atgcccgcga ccccatcttc    780
   ggcaaccaga tcatccccga caccgctatc ctcagcgtgg tgccatttca ccacggcttc    840
   ggcatgttca ccacgctggg ctacttgatc tgcggctttc gggtcgtgct catgtaccgc    900
   ttcgaggagg agctattctt gcgcagcttg caagactata agattcaatc tgccctgctg    960
   gtgcccacac tatttagctt cttcgctaag agcactctca tcgacaagta cgacctaagc   1020
   aacttgcacg agatcgccag cggcggggcg ccgctcagca aggaggtagg tgaggccgtg   1080
   gccaaacgct tccacctacc aggcatccgc cagggctacg gcctgacaga aacaaccagc 1140
   gccattctga tcacccccga aggggacgac aagcctggcg cagtaggcaa ggtggtgccc 1200
   ttcttcgagg ctaaggtggt ggacttggac accggtaaga cactgggtgt gaaccagcgc 1260
   ggcgagctgt gcgtccgtgg ccccatgatc atgagcggct acgttaacaa ccccgaggct 1320
   acaaacgctc tcatcgacaa ggacggctgg ctgcacagcg gcgacatcgc ctactgggac 1380
   gaggacgagc acttcttcat cgtggaccgg ctgaagagcc tgatcaaata caagggctac 1440
   caggtagccc cagccgaact ggagagcatc ctgctgcaac accccaacat cttcgacgcc 1500
   ggggtcgccg gcctgcccga cgacgatgcc ggcgagctgc ccgccgcagt cgtcgtgctg 1560
   gaacacggta aaaccatgac cgagaaggag atcgtggact atgtggccag ccaggttaca 1620
   accgccaaga agctgcgcgg tggtgttgtg ttcgtggacg aggtgcctaa aggactgacc 1680
   ggcaagttgg acgcccgcaa gatccgcgag attctcatta aggccaagaa gggcggcaag 1740
   atcgccgtgt aataattcta gagtcggggc ggccggccgc ttcgagcaga catgataaga 1800
   tacattgatg agtttggaca aaccacaact agaatgcagt gaaaaaaatg ctttatttgt 1860
   gaaatttgtg atgctattgc tttatttgta accattataa gctgcaataa acaagttaac 1920
   aacaacaatt gcattcattt tatgtttcag gttcaggggg aggtgtggga ggttttttaa 1980
   agcaagtaaa acctctacaa atgtggtaaa atcgataagg atccgtcgac cgatgccctt 2040
   gagagccttc aacccagtca gctccttccg gtgggcgcgg ggcatgacta tcgtcgccgc 2100
   acttatgact gtcttcttta tcatgcaact cgtaggacag gtgccggcag cgctcttccg 2160
   cttcctcgct cactgactcg ctgcgctcgg tcgttcggct gcggcgagcg gtatcagctc 2220
   actcaaaggc ggtaatacgg ttatccacag aatcagggga taacgcagga aagaacatgt 2280
   gagcaaaagg ccagcaaaag gccaggaacc gtaaaaaggc cgcgttgctg gcgtttttcc 2340
   ataggctccg cccccctgac gagcatcaca aaaatcgacg ctcaagtcag aggtggcgaa 2400
   acccgacagg actataaaga taccaggcgt ttccccctgg aagctccctc gtgcgctctc 2460
   ctgttccgac cctgccgctt accggatacc tgtccgcctt tctcccttcg ggaagcgtgg 2520
   cgctttctca tagctcacgc tgtaggtatc tcagttcggt gtaggtcgtt cgctccaagc 2580
   tgggctgtgt gcacgaaccc cccgttcagc ccgaccgctg cgccttatcc ggtaactatc 2640
   gtcttgagtc caacccggta agacacgact tatcgccact ggcagcagcc actggtaaca 2700
   ggattagcag agcgaggtat gtaggcggtg ctacagagtt cttgaagtgg tggcctaact 2760
   acggctacac tagaagaaca gtatttggta tctgcgctct gctgaagcca gttaccttcg 2820
   gaaaaagagt tggtagctct tgatccggca aacaaaccac cgctggtagc ggtggttttt 2880
   ttgtttgcaa gcagcagatt acgcgcagaa aaaaaggatc tcaagaagat cctttgatct 2940
   tttctacggg gtctgacgct cagtggaacg aaaactcacg ttaagggatt ttggtcatga 3000
   gattatcaaa aaggatcttc acctagatcc ttttaaatta aaaatgaagt tttaaatcaa 3060
   tctaaagtat atatgagtaa acttggtctg acagcggccg caaatgctaa accactgcag 3120
   tggttaccag tgcttgatca gtgaggcacc gatctcagcg atctgcctat ttcgttcgtc 3180
   catagtggcc tgactccccg tcgtgtagat cactacgatt cgtgagggct taccatcagg 3240
   ccccagcgca gcaatgatgc cgcgagagcc gcgttcaccg gcccccgatt tgtcagcaat 3300
   gaaccagcca gcagggaggg ccgagcgaag aagtggtcct gctactttgt ccgcctccat 3360
   ccagtctatg agctgctgtc gtgatgctag agtaagaagt tcgccagtga gtagtttccg 3420
   aagagttgtg gccattgcta ctggcatcgt ggtatcacgc tcgtcgttcg gtatggcttc 3480
   gttcaactct ggttcccagc ggtcaagccg ggtcacatga tcacccatat tatgaagaaa 3540
   tgcagtcagc tccttagggc ctccgatcgt tgtcagaagt aagttggccg cggtgttgtc 3600
   gctcatggta atggcagcac tacacaattc tcttaccgtc atgccatccg taagatgctt 3660
   ttccgtgacc ggcgagtact caaccaagtc gttttgtgag tagtgtatac ggcgaccaag 3720
   ctgctcttgc ccggcgtcta tacgggacaa caccgcgcca catagcagta ctttgaaagt 3780
   gctcatcatc gggaatcgtt cttcggggcg gaaagactca aggatcttgc cgctattgag 3840
   atccagttcg atatagccca ctcttgcacc cagttgatct tcagcatctt ttactttcac 3900
   cagcgtttcg ggtgtgcaaa aacaggcaag gcaaaatgcc gcaaagaagg gaatgagtgc 3960
   gacacgaaaa tgttggatgc tcatactcgt cctttttcaa tattattgaa gcatttatca 4020
   gggttactag tacgtctctc aaggataagt aagtaatatt aaggtacggg aggtattgga 4080
   caggccgcaa taaaatatct ttattttcat tacatctgtg tgttggtttt ttgtgtgaat 4140
   cgatagtact aacatacgct ctccatcaaa acaaaacgaa acaaaacaaa ctagcaaaat 4200
   aggctgtccc cagtgcaagt gcaggtgcca gaacatttct ct 4242
<210> 10
   <211> 62
   <212> DNA
   <213> mammalian
<400> 10
   ctgacatgcc caggcatgtc ttgacatgcc caggcatgtc ttgacatgcc caggcatgtc 60
   ta 62
<210> 11
   <211> 70
   <212> DNA
   <213> mammalian
<400> 11
   gatctagaca tgcctgggca tgtcaagaca tgcctgggca tgtcaagaca tgcctgggca 60
   tgtcaggtac 70
<210> 12
   <211> 23
   <212> DNA
   <213> Artificial
<220>
   <223> primer
<400> 12
   cttaatgttt ttggcatctt cca 23
<210> 13
   <211> 198
   <212> DNA
   <213> Simian virus 40
<400> 13
   tgcatctcaa ttagtcagca accatagtcc cgcccctaac tccgcccatc ccgcccctaa 60
   ctccgcccag ttccgcccat tctccgcccc atcgctgact aatttttttt atttatgcag 120
   aggccgaggc cgcctcggcc tctgagctat tccagaagta gtgaggaggc ttttttggag 180
   gcctaggctt ttgcaaaa 198
<210> 14
   <211> 181
   <212> DNA
   <213> herpes simplex virus 7
<400> 14
   taagaaaata tatttgcatg tctttagttc tatgatgaca caaaccccgc ccagcgtctt 60
   gtcattggcg aattcgaaca cgcagatgca gtcggggcgg cgcggtccca ggtccacttc 120
   gcatattaag gtgacgcgtg tggcctcgaa caccgagcga ccctgcagcg acccgcttaa 180
   a 181
<210> 15
   <211> 52
   <212> DNA
   <213> eucaryote
<400> 15
   gatctcgact ataaagaggg caggctgtcc tctaagcgtc accacgactt ca 52
<210> 16
   <211> 52
   <212> DNA
   <213> eucaryote
<400> 16
   agcttgaagt cgtggtgacg cttagaggac agcctgccct ctttatagtc ga 52
<210> 17
   <211> 18
   <212> DNA
   <213> Artificial
<220>
   <223> primer
<400> 17
   acacgcttcc ctggattg 18
<210> 18
   <211> 27
   <212> DNA
   <213> Artificial
<220>
   <223> primer
<400> 18
   ctgtcagtgg ggaacaagaa gtggaga 27
<210> 19
   <211> 1776
   <212> DNA
   <213> Artificial
<220>
   <223> luc2P gene
<400> 19
   atggaagatg ccaaaaacat taagaagggc ccagcgccat tctacccact cgaagacggg 60
   accgccggcg agcagctgca caaagccatg aagcgctacg ccctggtgcc cggcaccatc 120
   gcctttaccg acgcacatat cgaggtggac attacctacg ccgagtactt cgagatgagc 180
   gttcggctgg cagaagctat gaagcgctat gggctgaata caaaccatcg gatcgtggtg 240
   tgcagcgaga atagcttgca gttcttcatg cccgtgttgg gtgccctgtt catcggtgtg 300
   gctgtggccc cagctaacga catctacaac gagcgcgagc tgctgaacag catgggcatc 360
   agccagccca ccgtcgtatt cgtgagcaag aaagggctgc aaaagatcct caacgtgcaa 420
   aagaagctac cgatcataca aaagatcatc atcatggata gcaagaccga ctaccagggc 480
   ttccaaagca tgtacacctt cgtgacttcc catttgccac ccggcttcaa cgagtacgac 540
   ttcgtgcccg agagcttcga ccgggacaaa accatcgccc tgatcatgaa cagtagtggc 600
   agtaccggat tgcccaaggg cgtagcccta ccgcaccgca ccgcttgtgt ccgattcagt 660
   catgcccgcg accccatctt cggcaaccag atcatccccg acaccgctat cctcagcgtg 720
   gtgccatttc accacggctt cggcatgttc accacgctgg gctacttgat ctgcggcttt 780
   cgggtcgtgc tcatgtaccg cttcgaggag gagctattct tgcgcagctt gcaagactat 840
   aagattcaat ctgccctgct ggtgcccaca ctatttagct tcttcgctaa gagcactctc 900
   atcgacaagt acgacctaag caacttgcac gagatcgcca gcggcggggc gccgctcagc 960
   aaggaggtag gtgaggccgt ggccaaacgc ttccacctac caggcatccg ccagggctac 1020
   ggcctgacag aaacaaccag cgccattctg atcacccccg aaggggacga caagcctggc 1080
   gcagtaggca aggtggtgcc cttcttcgag gctaaggtgg tggacttgga caccggtaag 1140
   acactgggtg tgaaccagcg cggcgagctg tgcgtccgtg gccccatgat catgagcggc 1200
   tacgttaaca accccgaggc tacaaacgct ctcatcgaca aggacggctg gctgcacagc 1260
   ggcgacatcg cctactggga cgaggacgag cacttcttca tcgtggaccg gctgaagagc 1320
   ctgatcaaat acaagggcta ccaggtagcc ccagccgaac tggagagcat cctgctgcaa 1380
   caccccaaca tcttcgacgc cggggtcgcc ggcctgcccg acgacgatgc cggcgagctg 1440
   cccgccgcag tcgtcgtgct ggaacacggt aaaaccatga ccgagaagga gatcgtggac 1500
   tatgtggcca gccaggttac aaccgccaag aagctgcgcg gtggtgttgt gttcgtggac 1560
   gaggtgccta aaggactgac cggcaagttg gacgcccgca agatccgcga gattctcatt 1620
   aaggccaaga agggcggcaa gatcgccgtg aattctcacg gcttccctcc cgaggtggag 1680
   gagcaggccg ccggcaccct gcccatgagc tgcgcccagg agagcggcat ggatagacac 1740
   cctgctgctt gcgccagcgc caggatcaac gtctaa 1776
<210> 20
   <211> 4370
   <212> DNA
   <213> Artificial
<220>
   <223> plasmid
<400> 20
   ggcctaactg gccggtacct gagctcgcta gcctcgagga tatcaagatc tggcctcggc     60
   ggccaagctt ggcaatccgg tactgttggt aaagccacca tggaagatgc caaaaacatt    120
   aagaagggcc cagcgccatt ctacccactc gaagacggga ccgccggcga gcagctgcac    180
   aaagccatga agcgctacgc cctggtgccc ggcaccatcg cctttaccga cgcacatatc    240
   gaggtggaca ttacctacgc cgagtacttc gagatgagcg ttcggctggc agaagctatg    300
   aagcgctatg ggctgaatac aaaccatcgg atcgtggtgt gcagcgagaa tagcttgcag    360
   ttcttcatgc ccgtgttggg tgccctgttc atcggtgtgg ctgtggcccc agctaacgac    420
   atctacaacg agcgcgagct gctgaacagc atgggcatca gccagcccac cgtcgtattc    480
   gtgagcaaga aagggctgca aaagatcctc aacgtgcaaa agaagctacc gatcatacaa    540
   aagatcatca tcatggatag caagaccgac taccagggct tccaaagcat gtacaccttc    600
   gtgacttccc atttgccacc cggcttcaac gagtacgact tcgtgcccga gagcttcgac    660
   cgggacaaaa ccatcgccct gatcatgaac agtagtggca gtaccggatt gcccaagggc    720
   gtagccctac cgcaccgcac cgcttgtgtc cgattcagtc atgcccgcga ccccatcttc 780
   ggcaaccaga tcatccccga caccgctatc ctcagcgtgg tgccatttca ccacggcttc 840
   ggcatgttca ccacgctggg ctacttgatc tgcggctttc gggtcgtgct catgtaccgc 900
   ttcgaggagg agctattctt gcgcagcttg caagactata agattcaatc tgccctgctg 960
   gtgcccacac tatttagctt cttcgctaag agcactctca tcgacaagta cgacctaagc 1020
   aacttgcacg agatcgccag cggcggggcg ccgctcagca aggaggtagg tgaggccgtg 1080
   gccaaacgct tccacctacc aggcatccgc cagggctacg gcctgacaga aacaaccagc 1140
   gccattctga tcacccccga aggggacgac aagcctggcg cagtaggcaa ggtggtgccc 1200
   ttcttcgagg ctaaggtggt ggacttggac accggtaaga cactgggtgt gaaccagcgc 1260
   ggcgagctgt gcgtccgtgg ccccatgatc atgagcggct acgttaacaa ccccgaggct 1320
   acaaacgctc tcatcgacaa ggacggctgg ctgcacagcg gcgacatcgc ctactgggac 1380
   gaggacgagc acttcttcat cgtggaccgg ctgaagagcc tgatcaaata caagggctac 1440
   caggtagccc cagccgaact ggagagcatc ctgctgcaac accccaacat cttcgacgcc 1500
   ggggtcgccg gcctgcccga cgacgatgcc ggcgagctgc ccgccgcagt cgtcgtgctg 1560
   gaacacggta aaaccatgac cgagaaggag atcgtggact atgtggccag ccaggttaca 1620
   accgccaaga agctgcgcgg tggtgttgtg ttcgtggacg aggtgcctaa aggactgacc 1680
   ggcaagttgg acgcccgcaa gatccgcgag attctcatta aggccaagaa gggcggcaag 1740
   atcgccgtga attctcacgg cttccctccc gaggtggagg agcaggccgc cggcaccctg 1800
   cccatgagct gcgcccagga gagcggcatg gatagacacc ctgctgcttg cgccagcgcc 1860
   aggatcaacg tctaaggccg cgactctaga gtcggggcgg ccggccgctt cgagcagaca 1920
   tgataagata cattgatgag tttggacaaa ccacaactag aatgcagtga aaaaaatgct 1980
   ttatttgtga aatttgtgat gctattgctt tatttgtaac cattataagc tgcaataaac 2040
   aagttaacaa caacaattgc attcatttta tgtttcaggt tcagggggag gtgtgggagg 2100
   ttttttaaag caagtaaaac ctctacaaat gtggtaaaat cgataaggat ccgtcgaccg 2160
   atgcccttga gagccttcaa cccagtcagc tccttccggt gggcgcgggg catgactatc 2220
   gtcgccgcac ttatgactgt cttctttatc atgcaactcg taggacaggt gccggcagcg 2280
   ctcttccgct tcctcgctca ctgactcgct gcgctcggtc gttcggctgc ggcgagcggt 2340
   atcagctcac tcaaaggcgg taatacggtt atccacagaa tcaggggata acgcaggaaa 2400
   gaacatgtga gcaaaaggcc agcaaaaggc caggaaccgt aaaaaggccg cgttgctggc 2460
   gtttttccat aggctccgcc cccctgacga gcatcacaaa aatcgacgct caagtcagag 2520
   gtggcgaaac ccgacaggac tataaagata ccaggcgttt ccccctggaa gctccctcgt 2580
   gcgctctcct gttccgaccc tgccgcttac cggatacctg tccgcctttc tcccttcggg 2640
   aagcgtggcg ctttctcata gctcacgctg taggtatctc agttcggtgt aggtcgttcg 2700
   ctccaagctg ggctgtgtgc acgaaccccc cgttcagccc gaccgctgcg ccttatccgg 2760
   taactatcgt cttgagtcca acccggtaag acacgactta tcgccactgg cagcagccac 2820
   tggtaacagg attagcagag cgaggtatgt aggcggtgct acagagttct tgaagtggtg 2880
   gcctaactac ggctacacta gaagaacagt atttggtatc tgcgctctgc tgaagccagt 2940
   taccttcgga aaaagagttg gtagctcttg atccggcaaa caaaccaccg ctggtagcgg 3000
   tggttttttt gtttgcaagc agcagattac gcgcagaaaa aaaggatctc aagaagatcc 3060
   tttgatcttt tctacggggt ctgacgctca gtggaacgaa aactcacgtt aagggatttt 3120
   ggtcatgaga ttatcaaaaa ggatcttcac ctagatcctt ttaaattaaa aatgaagttt 3180
   taaatcaatc taaagtatat atgagtaaac ttggtctgac agcggccgca aatgctaaac 3240
   cactgcagtg gttaccagtg cttgatcagt gaggcaccga tctcagcgat ctgcctattt 3300
   cgttcgtcca tagtggcctg actccccgtc gtgtagatca ctacgattcg tgagggctta 3360
   ccatcaggcc ccagcgcagc aatgatgccg cgagagccgc gttcaccggc ccccgatttg 3420
   tcagcaatga accagccagc agggagggcc gagcgaagaa gtggtcctgc tactttgtcc 3480
   gcctccatcc agtctatgag ctgctgtcgt gatgctagag taagaagttc gccagtgagt 3540
   agtttccgaa gagttgtggc cattgctact ggcatcgtgg tatcacgctc gtcgttcggt 3600
   atggcttcgt tcaactctgg ttcccagcgg tcaagccggg tcacatgatc acccatatta 3660
   tgaagaaatg cagtcagctc cttagggcct ccgatcgttg tcagaagtaa gttggccgcg 3720
   gtgttgtcgc tcatggtaat ggcagcacta cacaattctc ttaccgtcat gccatccgta 3780
   agatgctttt ccgtgaccgg cgagtactca accaagtcgt tttgtgagta gtgtatacgg 3840
   cgaccaagct gctcttgccc ggcgtctata cgggacaaca ccgcgccaca tagcagtact 3900
   ttgaaagtgc tcatcatcgg gaatcgttct tcggggcgga aagactcaag gatcttgccg 3960
   ctattgagat ccagttcgat atagcccact cttgcaccca gttgatcttc agcatctttt 4020
   actttcacca gcgtttcggg gtgtgcaaaa acaggcaagc aaaatgccgc aaagaaggga 4080
   atgagtgcga cacgaaaatg ttggatgctc atactcgtcc tttttcaata ttattgaagc 4140
   atttatcagg gttactagta cgtctctcaa ggataagtaa gtaatattaa ggtacgggag 4200
   gtattggaca ggccgcaata aaatatcttt attttcatta catctgtgtg ttggtttttt 4260
   gtgtgaatcg atagtactaa catacgctct ccatcaaaac aaaacgaaac aaaacaaact 4320
   agcaaaatag gctgtcccca gtgcaagtgc aggtgccaga acatttctct 4370

## Description

The present invention relates to a mutagenicity test method for confirmation of mutagenicity and carcinogenicity of a test substance on a mammalian cell, and to a use of a transformant for the method.

Chemical substances with which humans come into contact, such as foods, cosmetics, and pharmaceuticals, and substances contained in products to be discarded into the environment are each required to have no carcinogenicity. In safety evaluation of those substances, it is essential to evaluate whether or not a substance to be used has mutagenicity because many mutagenic substances have carcinogenicity.

The Ames test using Salmonella, which is adopted under the Law concerning Examination and Regulation of Manufacture, etc. of Chemical Substances, the Pharmaceutical Affairs Law, or the like, is most generally used as the existing mutagenicity test. This test is a test using a microorganism. Accordingly, a chemical substance assessed to be positive for mutagenicity in the test does not necessarily give a similar result in a human. Meanwhile, a test using a culture cell of a mammal and a test using an animal individual, such as a mouse lymphoma TK test, a chromosome aberration test, and a micronucleus test, are also established as mutagenicity test methods, but involve complicated operations and require at least 2 days or more for obtaining results.

Under such circumstances, the inventors of the present invention developed a mutagenicity test method including the steps of: (1) bringing a transformant harboring a recombinant vector including a p53 binding sequence, a minimal promoter that can function in a mammalian cell, and a reporter gene linked so that the gene can be expressed by the minimal promoter into contact with a test substance; and (2) assessing whether or not the test substance affects an expression amount of the reporter gene in the transformant by comparing an expression amount of the reporter gene in the transformant brought into contact with the test substance to an expression amount of the reporter gene in the transformant not brought into contact with the test substance (Patent Literature 1).

### Patent Literature

[PTL 1] JP 4243716 B2
Moreover, Ohno et al. Mutation Research, 656, 2008, 27-35 describes a genotoxicity test system based on p53R2 gene expression in human cells. Mizota et al. Mutation Research, 724, 2011, 76-85 describes validation of a genotoxicity test based on p53R2 gene expression in human lymphoblastoid cells.

A main object of the present invention is to provide a test method that allows mutagenicity on a mammal including a human to be confirmed accurately and simply in a short time.

The inventors of the present invention have made intensive studies in order to achieve the object. As a result, the inventors have found that a mutagenicity test can be carried out by a mutagenicity test method including the step of culturing a transformant harboring a recombinant vector including a p53 binding sequence, a minimal promoter, and a reporter gene while bringing the transformant into contact with a test substance for a shorter time than ever before. In particular, the inventors have found that the use of a specific combination of the recombinant vector with a host cell allows a time for contact with the test substance to be shortened to a great extent. The present invention has been completed based on the findings.

Thus, the present invention provides the following items.

Item 1. A mutagenicity test method, including the steps of:
(1) culturing a transformant harboring a recombinant vector including a p53 binding sequence, a minimal promoter that functions in a mammalian cell, and a reporter gene linked so that the gene is expressed by the minimal promoter, in the presence of a test substance for from 4 to 10 hours; and
(2) assessing whether or not the test substance affects an expression amount of the reporter gene in the transformant by comparing an expression amount of the reporter gene in the transformant cultured in the step (1) to an expression amount of the reporter gene in the transformant free of contact with the test substance, wherein the reporter gene is luc2, and the transformant is a TK6 cell transfected with the recombinant vector.

Item 2. The mutagenicity test method according to Item 1, in which the minimal promoter includes an SV40 promoter.

Item 3. The mutagenicity test method according to Item 1 or 2, in which the p53 binding sequence, the minimal promoter, and the reporter gene are disposed in the recombinant vector in an order of the p53 binding sequence, the minimal promoter, and the reporter gene from an upstream side of the recombinant vector.

Item 4. The mutagenicity test method according to any one of Items 1 to 3, in which the p53 binding sequence includes a base sequence set forth in SEQ ID NO: 1:
5'-WRRCWWGYYY-3': SEQ ID NO: 1
in SEQ ID NO: 1, R's each represent a nucleotide including a purine base, W's each represent a nucleotide including adenine or thymine, and Y's each represent a nucleotide including a pyrimidine base.

Item 5. The mutagenicity test method according to any one of Items 1 to 4, in which the p53 binding sequence includes a base sequence set forth in SEQ ID NO: 2:
5'-WRRCWWGYYY WRRCWWGYYY-3': SEQ ID NO: 2
in SEQ ID NO: 2, R's each represent a nucleotide including a purine base, W's each represent a nucleotide including adenine or thymine, and Y's each represent a nucleotide including a pyrimidine base.

Item 6. The mutagenicity test method according to Item 1, in which the expression amount of the reporter gene is a relative value to that of a control gene.

Item 7. Use of a transformant for the mutagenicity test according to Items 1 to 6, wherein the transformant is a TK6 cell harboring a recombinant vector including a p53 binding sequence, a minimal promoter that functions in a mammalian cell, and a reporter gene linked so that the gene is expressed by the minimal promoter, wherein the reporter gene is luc 2.

Item 8. Use of a kit for a mutagenicity test according to Items 1 to 6, wherein the kit includes the transformant according to Item 7.

According to one embodiment of the present invention, the mutagenicity on a mammal including a human can be confirmed more accurately and simply than ever before.

Moreover, the mutagenicity test method of the present invention does not directly evaluate a change in phenotype due to DNA damage but evaluates a biological reaction due to DNA damage. Thus, the DNA damage can be detected with high sensitivity.

In addition, unlike related-art mutagenicity tests, the method of the present invention does not utilize a microorganism but utilizes a reaction system for DNA damage on a cell of a mammal including a human. Thus, its test results accurately reflect the mutagenicity on a human.

In addition, according to one embodiment of the present invention, a time for contact between the transformant and the test substance can be made shorter than ever before. Thus, the whole test can be performed within about 1 day, i.e., a time for the whole test can be shortened to a great extent.

FIG. 1 shows a base sequence of a pGL4.10 plasmid (Sequence of pGL4.10 plasmid).
FIG. 2 shows a base sequence of the pGL4.10 plasmid.
FIG. 3 is a graph showing luciferase activities in bringing each of a pGL4-p53R2×3-SV40-Luc2 transformant and a PGV-p53R2x3-Luc transformant into contact with adriamycin in a mutagenicity test method of Example 5 of the present invention. DMSO represents a group in which only the solvent is added, ADM 0.003, ADM 0.01, ADM 0.03, ADM 0.1, ADM 0.2, and ADM 0.7 represent groups in which adriamycin is added at 0.003 µg/mL, 0.01 µg/mL, 0.03 µg/mL, 0.1 µg/mL, 0.2 µg/mL, and 0.7 µg/mL, respectively.
FIG. 4 is a graph showing luciferase activities of pGL4-p53R2x3-SV40-Luc2 transformants of various human-derived cells of Example 6 at different concentrations of adriamycin.
FIG. 5 is a graph showing luciferase activities of PGV-p53R2x3-Luc transformants of various human-derived cells of Reference Example 1 at different concentrations of adriamycin.
FIG. 6 is a graph showing luciferase activities of PGV-p53R2x3-Luc transformants of various human-derived cells of Reference Example 2 at different concentrations of adriamycin.
FIG. 7 shows a base sequence of a pGL4.11 plasmid (Sequence of pGL4.11 plasmid).

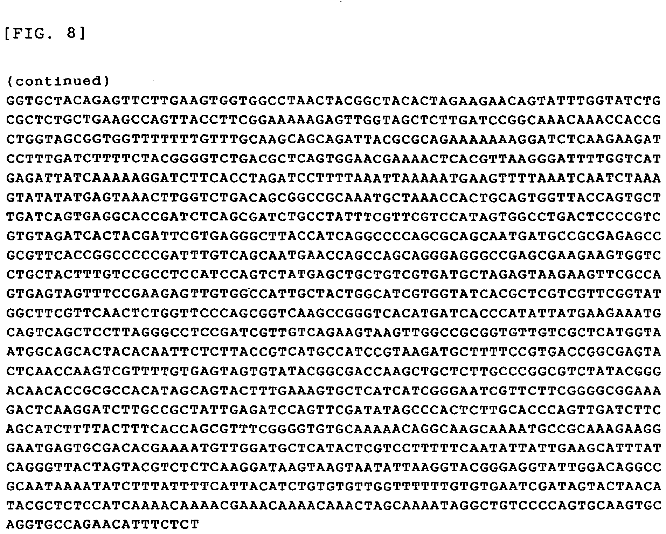

FIG. 8 shows a base sequence of the pGL4.11 plasmid.
FIG. 9 is a graph showing a time-dependent change in luciferase activity in bringing a pGL4-p53R2x3-Luc2P stable transformant into contact with adriamycin in a mutagenicity test method of Example 9.
FIG. 10 is a graph showing luciferase activities in bringing a pGL4-p53R2x3-Luc2P stable transformant into contact with different concentrations of adriamycin in a mutagenicity test method of Example 10.
FIG. 11 is a graph showing luciferase activities corrected or not corrected with an internal control in bringing a stable transformant of Example 12 into contact with 5-aza-dC.

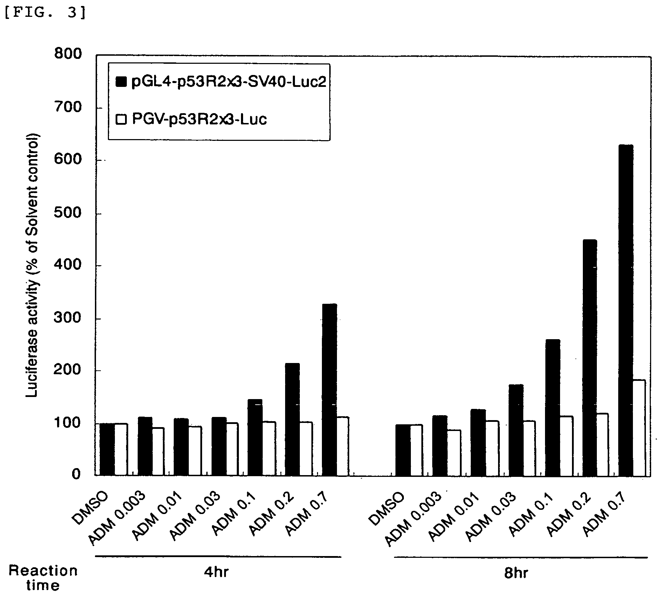

In FIGS. 3, 4, 5, 6, 9, 10, and 11, the ordinate axis represents "Luciferase activity (% of Solvent control)," that is, a measured value for luciferase activity, which is expressed as a relative value to the luciferase activity of a solvent control sample as a control.

Hereinafter, the present invention is described in detail.

### (I) Recombinant vector

A transformant to be used in a method of the present invention harbors a recombinant vector including a p53 binding sequence, a minimal promoter that can function in a mammalian cell, and a reporter gene linked so that the gene can be expressed by the minimal promoter.

### p53 binding sequence

A p53 protein is a transcription factor that is activated by phosphorylation at a specific site at the time of DNA damage by a chemical substance, UV light, γ-rays, or the like, and binds to transcriptional regulatory regions of a specific group of genes, thereby exhibiting functions of regulating the transcription thereof.

The amino acid sequence of the p53 protein is known. For example, the amino acid sequence of a human (*Homo sapiens*) p53 protein is described in GenBank accession No. NP_001119584, and the amino acid sequence of a mouse (*Mus musculus*) p53 protein is described in GenBank accession No. NP_035770.

An example of the p53 binding sequence is a DNA sequence present in a transcriptional regulatory region upstream or within introns of a group of target genes whose transcriptional activity is regulated by p53 owing to DNA damage in a mammalian cell.

It is preferred to use p53 binding sequences of a group of genes whose transcription is activated to show an increase in transcription amount when an active form of p53 phosphorylated by DNA damage recognizes and binds to their transcriptional regulatory regions. Specific examples of the genes whose transcription is activated by p53 include: p53R2 involved in DNA repair; p21/Waf-1 involved in the regulation of a cell cycle; and p53AIP1, Bax, and p53DINP1 involved in the regulation of apoptosis.

p53 binding DNA sequences of a group of genes whose transcription is promoted byp53 in a natural mammalian cell generally include a sequence 5'-WRRCWWGYYY WRRCWWGYYY-3' (SEQ ID NO: 2: R's each represent a nucleotide having a purine base, W's each represent a nucleotide including adenine or thymine, and Y's each represent a nucleotide including a pyrimidine base) including two repeats of a consensus sequence 5'-WRRCWWGYYY-3' (SEQ ID NO: 1: R's each represent a nucleotide having a purine base, W's each represent a nucleotide including adenine or thymine, and Y's each represent a nucleotide including a pyrimidine base).

Accordingly, the recombinant vector to be used in the present invention preferably includes, as the p53 binding sequence, one or more copies of the consensus sequence of SEQ ID NO: 1. In addition, the recombinant vector to be used in the present invention preferably includes, as the p53 binding sequence, one or more copies of the consensus sequence of SEQ ID NO: 2. The recombinant vector may include, as the p53 binding sequence, one or more copies of a base sequence having substitutions of up to about several, in particular, one or two nucleotides in the base sequence of SEQ ID NO: 2.

The recombinant vector to be used in the present invention more preferably includes, as the p53 binding sequence, about three copies of the base sequence of SEQ ID NO: 2 or a base sequence having substitutions of up to several nucleotides in the base sequence of SEQ ID NO: 2 (preferably a base sequence of SEQ ID NO: 3 or a base sequence having substitutions of up to several nucleotides in the base sequence of SEQ ID NO: 3). When two or more copies of the consensus sequence of SEQ ID NO: 2 are included, they may be directly linked or may have from about 1 to 20 nucleotides sandwiched therebetween.

In a natural mammalian cell, 5 '-TGACATGCCCAGGCATGTCT-3' (SEQ ID NO: 3: Nature, 404, 42-49 (2000)) is present in intron 1 of p53R2, 5'-GGGCAGGCCCGGGCTTGTCG-3' (SEQ ID NO: 4: Oncogene, 21, 990-999 (2002)) is present in intron 1 of Bax, 5'-TCTCTTGCCCGGGCTTGTCG-3' (SEQ ID NO: 5: Cell, 102, 849-862 (2000)) is present in intron 1 of p53AIP1, 5'-GAACTTGGGGGAACATGTTT-3' (SEQ ID NO: 6: Mol. Cell, 8, 85-94 (2001)) is present in intron 2 of p53DINP1, and 5'-GAACATGTCCCAACATGTTG-3' (SEQ ID NO: 7: Cell, 75, 817-825 (1993)) is present upstream of p21/Waf-1.

In the present invention, the p53 binding sequence is preferably SEQ ID NO: 3 included in the above-mentioned consensus sequence (SEQ ID NO: 2). However, a sequence including one or more copies of the base sequence of SEQ ID NO: 4, 5, 6, or 7 (in particular, the base sequence of SEQ ID NO: 5) may also be used. In addition, a sequence including one or more copies of a base sequence having substitutions of about one to three nucleotides in the base sequence of SEQ ID NO: 3, 4, 5, 6, or 7 may be used as long as the sequence corresponds to the base sequence of SEQ ID NO: 2. In particular, a sequence including three copies of the base sequence of SEQ ID NO: 3, 4, 5, 6, or 7 (in particular, the base sequence of SEQ ID NO: 3 or 5) is preferred. In addition, when a plurality of copies of the base sequence of SEQ ID NO: 3, 4, 5, 6, or 7 are included, they may be included in combination. An example thereof is a base sequence in which the sequence of SEQ ID NO: 3, the sequence of SEQ ID NO: 4, and the sequence of SEQ ID NO: 5 are linked.

A DNA sequence including a consensus sequence may be chemically synthesized, or may be prepared by amplification by PCR using a primer designed based on the consensus sequence using chromosomal DNA of a mammal as a template, followed by cloning.

In the present invention, p53 binding sequences of a group of genes whose transcription amount is reduced by the binding of p53 may also be used.

In addition, in the recombinant vector to be used in the present invention, in place of the p53 binding sequence, a binding sequence of any other transcription factor to be activated owing to DNA damage may be used. That is, transcription factor binding sequences of a group of other target genes whose transcriptional activity is regulated owing to DNA damage in a mammalian cell may also be used. An example of such target genes is a gene encoding a kinase protein ATM or ATR. ATM is a kinase that phosphorylates p53 in response to DNA damage. Thus, a transcription factor binding sequence present in the upstream region of an ATM gene may also be used in place of the p53 binding sequence. In addition, ATR is a kinase that phosphorylates p53 in response to DNA damage mainly by UV light or the like. Thus, a transcription factor binding sequence present in the upstream region of an ATM gene may also be used in place of the p53 binding sequence.

### Minimal promoter

A minimal promoter that can be expressed in a mammalian cell is also called a core promoter, and is generally a region found in a relatively narrow portion around the transcriptional start site of a gene. Inaddition, the minimal promoter is a main functional element involved in transcriptional regulation, and is a sequence that does not include any other functional element involved in transcriptional regulation, such as a recognition sequence of another transcriptional regulatory factor, or a sequence that includes such functional element, in which the element does not essentially change a transcriptional regulatory function to be exhibited by the p53 binding sequence and the minimal promoter.

In the recombinant vector to be used in the present invention, a known minimal promoter in a mammal may be used without any limitation. A base sequence of such minimal promoter is exemplified by: a TATA box, which is a DNA region that determines the transcriptional start site in transcription by RNA polymerase II and is involved in the maintenance of the minimum transcriptional level; and a base sequence in the vicinity of the transcriptional start site. Specific examples thereof include: a base sequence in the 5'-upstream region of a mouse metallothionein I gene (Genbank Accession No. J00605) ; a base sequence in the 5'-upstream region of a chicken ovalbumin gene (Genbank accession No. J00895) ; a base sequence in the 5'-upstream region of a herpes simplex virus (HSV)-derived thymidine kinase (tk) gene (Proc. Natl. Acad. Sci. USA., 78, 1441-1445 (1981)); and a minimal promoter of a simian virus (SV40). Those minimal promoter sequences are each preferably used as the minimal promoter in the recombinant vector used in the present invention. Of the minimal promoters, SV40 is particularly preferred. In addition, DNA sequences having additions, deletions, or substitutions of about one or several nucleotides in those DNA sequences may be used as long as the sequences each function as a minimal promoter in a mammalian cell.

In addition, the recombinant vector to be used in the present invention may include a wider range of a promoter region including the minimal promoter as long as the transcriptional regulatory function to be exhibited by the p53 binding sequence and the minimal promoter is not essentially changed.

DNAs formed of those sequences may be prepared by, for example, chemical synthesis based on base sequences thereof.

### Reporter gene

In the present invention, the reporter gene refers to a gene whose expression can be qualitatively or quantitatively confirmed. In particular, a gene whose expression amount can be measured based on the enzymatic activity or the like of its transcriptional product (reporter protein) is preferred from the viewpoint that the measurement of the expression amount is easy. Examples of such reporter gene include: a gene encoding an enzyme protein such as firefly (*Photinus pyralis*) luciferase, Sea pansy (*Renilla reniformis*) luciferase, β-galactosidase, chloramphenicol acetyltransferase, alkaline phosphatase, or β-glucuronidase; and a gene encoding a photoprotein such as a green fluorescent protein (GFP).

As those reporter genes, one contained in a commercially available reporter plasmid may be used. The reporter gene used in the present invention is a luc2 gene. The luc2 gene is a gene encoding engineered luciferase derived from firefly luciferase.

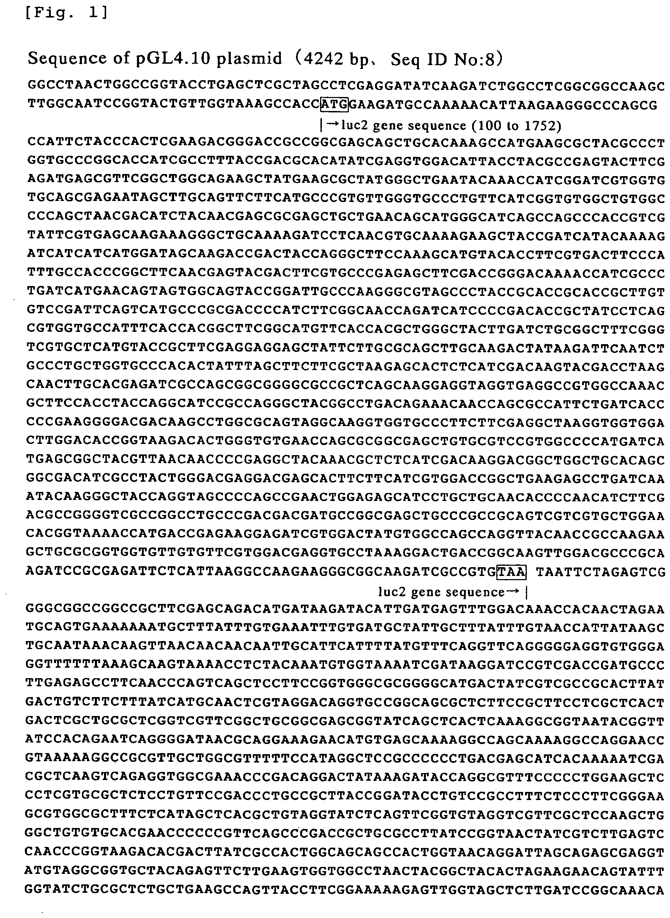

In the present invention, the luc2 gene is not particularly limited as long as the gene is formed of a base sequence encoding a protein having luciferase activity. The luc2 gene includes not only a base sequence (SEQ ID NO: 8) corresponding to a portion indicated by "luc2 gene sequence" in the plasmid shown in FIG. 1 but also a base sequence having additions, deletions, or substitutions of about one or several nucleotides in the base sequence set forth in SEQ ID NO: 8. In addition, the luc2 gene also includes a base sequence encoding an amino acid sequence in which a tag sequence, a signal sequence, or the like is added to the N-terminus or C-terminus of an amino acid sequence encoded by the base sequence set forth in SEQ ID NO: 8. A specific example thereof also includes a luc2P gene, which is an engineered gene obtained by such engineering that the intracellular accumulation amount of a luciferase protein is reduced through the addition of a protein degradation sequence (hPEST) to the C-terminus.

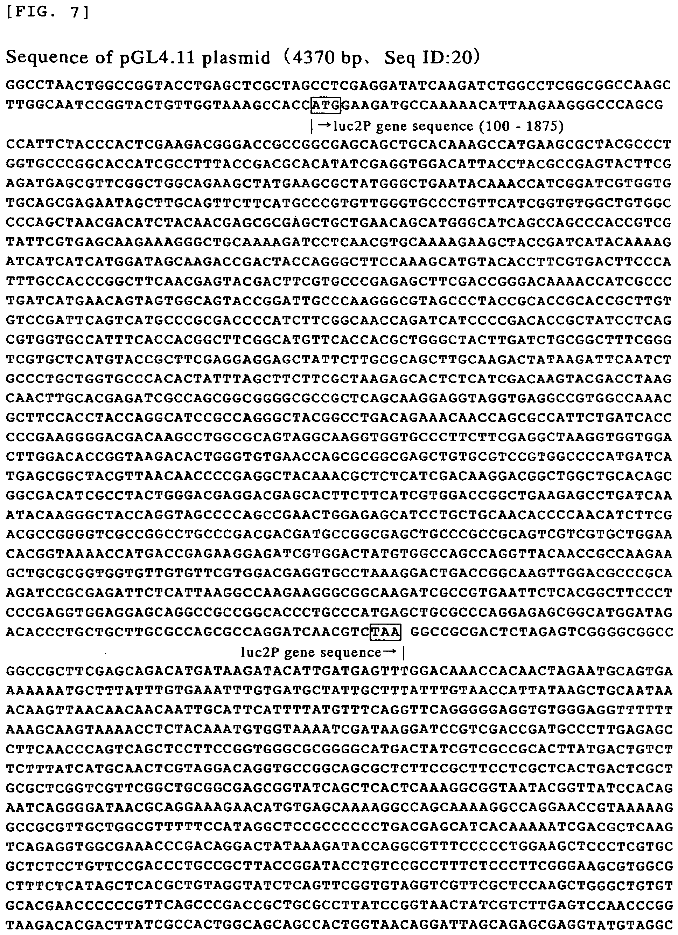

The luc2P gene includes not only a base sequence (SEQ ID NO: 19) corresponding to a portion indicated by "luc2P gene sequence" in the plasmid shown in FIG. 7 but also a base sequence having additions, deletions, or substitutions of about one or several nucleotides in the base sequence set forth in SEQ ID NO: 19, the base sequence encoding a protein having luciferase activity.

In addition, in the present invention, the luc2 gene also includes the base sequence set forth in SEQ ID NO: 8 or SEQ ID NO: 19 engineered so as to further increase the expression level of luciferase.

In this context, the expression level of luciferase (e.g., firefly luciferase encoded by the base sequence set forth in SEQ ID NO: 8 or SEQ ID NO: 19 and its engineered sequence) may be, for example, evaluated by: first transfecting host cells (e.g., mammalian cells to be described later) with a reporter plasmid including a luciferase gene; adding a positive control substance (e.g., adriamycin); then measuring the expression amount of the luciferase gene; subsequently measuring the expression amount of the luciferase gene at a basal level in the same manner as described above except that the positive control substance is not added; and determining a ratio (S/N ratio) between these expression amounts. More specifically, for example, first, host cells transiently or stably transfected with a reporter plasmid including a luciferase gene are brought into contact with a positive control substance and then washed with PBS (-) or the like. The cells are lysed with addition of a cell lysis agent containing a surfactant. A luciferase substrate (e.g., luciferin when the luciferase, is firefly luciferase) is added to the cell lysate, and a light emission amount based on the degradation of the substrate is quantified using a luminometer (positive control substance addition group) . Next, the expression amount of the luciferase gene at a basal level is quantified in the same manner as described above except that the positive control substance is not added (control group). The expression level of luciferase may be evaluated by calculating a ratio of the light emission amount (expression amount) in the positive control substance addition group to that in the control group. When the S/N ratio in the case of using engineered luciferase is higher than the S/N ratio in the case of using luciferase, the expression level can be evaluated to have been increased by the engineering.

### Cell selection marker gene

The recombinant vector to be used in the present invention may include a cell selection marker gene that can function in a mammalian cell so that the harboring of the recombinant vector in cells can be confirmed. The term "cell selection marker gene" refers to a gene encoding a phenotypic character that can serve as a marker in distinguishing cells transformed with DNA including the gene from non-transformed cells. The phrase "can function in an animal cell" means that the character can be expressed in an animal cell, and the term "gene that can function in an animal cell" refers to a gene that is under the control of a promoter having a transcriptional start ability in an animal cell and hence is capable of being expressed in the animal cell.

The cell selection marker gene effective in an animal cell may be exemplified by a gene that can impart resistance to a drug for suppressing or inhibiting the growth of an animal cell to the cell. Specific examples thereof include a neomycin resistance-imparting gene (aminoglycoside phosphotransferase gene), a hygromycin resistance-imparting gene (hygromycin phosphotransferase gene), and a blasticidin S resistance-imparting gene (blasticidin S deaminase gene). Of those, a blasticidin S resistance-imparting gene is preferred from the viewpoint that a transformed cell can be selected in a shorter period.

### Structure

The recombinant vector to be used in the present invention includes a p53 binding sequence, a minimal promoter that can function in a mammalian cell, and a reporter gene linked so that the gene can be expressed by the minimal promoter. That is, the reporter gene is linked so that the gene can be expressed downstream of a transcriptional regulatory region including the p53 binding sequence and the minimal promoter. Any of the transcription factor binding sequence and the minimal promoter may be present more upstream. The recombinant vector may be, for example, a vector in which the luc2 gene sequence described above as the reporter gene is linked.

About one or several nucleotides may be present between the p53 binding sequence and the minimal promoter. In addition, about one or several nucleotides may be present between the transcriptional regulatory region including the p53 binding sequence and the minimal promoter, and the reporter gene in such a range that the reporter gene can be expressed.

A base sequence encoding a peptide to be expressed together with a reporter protein (i.e., expressed as a fusion protein) may be present upstream or downstream of the reporter gene. Such peptide is exemplified by: a tag sequence such as an His-tag, an myc-tag, an FLAG-tag, or an HA-tag; and a signal sequence such as a recombinant vector including a p53 binding sequence, a minimal promoter that can function in a mammalian cell, and a reporter gene linked so that the gene can be expressed by the minimal promoter (e.g., an hPEST sequence), a nuclear localization signal sequence (for example, an NLS sequence), or a secretion signal sequence.

In order to realize such vector, the recombinant vector to be used in the present invention may be obtained by: utilizing a commercially available expression vector for mammalian cells having a minimal promoter and a gene insertion site downstream thereof; and inserting the p53 binding sequence and the reporter gene into the vector. Examples of such commercially available expression vector for mammalian cells include pUC18 and pUC19. In addition, as described above, the recombinant vector to be used in the present invention may be obtained by inserting the p53 binding sequence into a vector commercially available as a commercially available reporter plasmid. Examples of the commercially available reporter plasmid include luciferase plasmids pGL4-Luc2, pGL4-Luc2P, pT811u, pSluc2, and pGL3. The recombinant vector to be used in the present invention is preferably a pGL4-Luc2 plasmid having inserted therein the p53 binding sequence, the minimal promoter, and the like, or a pGL4-Luc2P plasmid having inserted therein the p53 binding sequence, the minimal promoter, and the like.

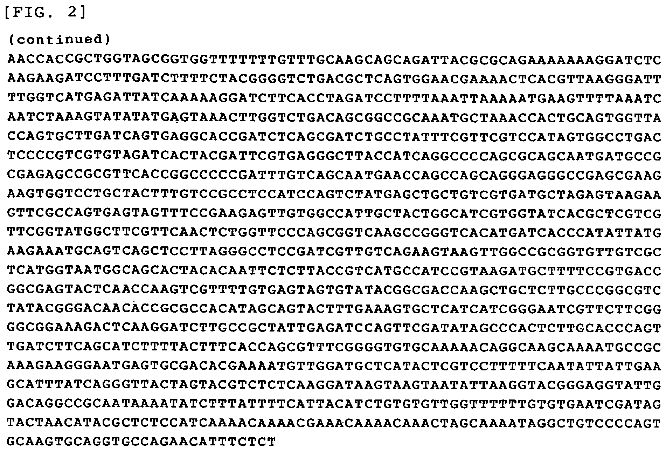

The pGL4-Luc2 plasmid and the pGL4-Luc2P plasmid are commercially available from Promega. The pGL4-Luc2 plasmid is, for example, formed of the base sequence set forth in SEQ ID NO: 9 (pGL4.10 plasmid). FIGS. 1 and 2 show the base sequence set forth in SEQ ID NO: 9. In addition, the pGL4-Luc2 plasmid to be used as a raw material for the recombinant vector in the present invention also includes a base sequence having additions, deletions, or substitutions of about one or several nucleotides in the base sequence set forth in SEQ ID NO: 9. In addition, the pGL4-Luc2P plasmid is, for example, formed of a base sequence set forth in SEQ ID NO: 20 (pGL4. 11 plasmid). FIG. 7 shows the base sequence set forth in SEQ ID NO: 20. In addition, the pGL4-Luc2P plasmid to be used as a raw material for the recombinant vector in the present invention also includes a base sequence having additions, deletions, or substitutions of about one or several nucleotides in the base sequence set forth in SEQ ID NO: 20. It should be noted that the pGL4-Luc2P plasmid is a plasmid including a base sequence encoding an hPEST sequence downstream of the coding region of a reporter protein in the pGL4-Luc2 plasmid. The plasmid encodes an engineered gene obtained by such engineering that the intracellular accumulation amount of a luciferase protein is reduced through the addition of a protein degradation sequence (hPEST) to the C-terminus of an amino acid sequence encoded by the base sequence set forth in SEQ ID NO: 8 (luc2P gene).

The recombinant vector to be used in the present invention desirably has a compact size, and for example, preferably has a size of from about 2 to 10 kb, from the viewpoints of ease of handling and a low incidence of gene recombination in vector molecules or between the molecules, dissociation from chromosome in stably transformed cells, or the like.

The recombinant vector to be used in the present invention is not limited to a full-length plasmid and may be a gene construct as a part thereof as long as the vector includes the minimal promoter that can function in a mammalian cell and the reporter gene linked so that the gene can be expressed by the minimal promoter.

The recombinant vector used in the present invention may be suitably used as a recombinant vector for producing a transformant to be subjected to a mutagenicity test to be described later.

### (II) Transformant

A transformant used in the present invention is a cell harboring the above-mentioned recombinant vector used in the present invention. The transformant used in the present invention may transiently harbor or stably harbor the recombinant vector.

### Mammalian cell

In the preparation of the transformant, host cells to be transfected with the recombinant vector are not particularly limited as long as the cells are mammalian. As the host cells, mammalian cells in which wild-type p53 is expressed and normally functions may be used, and abnormal cells having a lowered, deleted, or mutated function of p53 may also be used by being separately transfected with a wild-type p53 gene so as to express p53. In addition, even in the cells expressing wild-type p53, for example, when its expression amount is small or when the promoter activity in the recombinant vector is too weak, the cells further separately transfected with the wild-type p53 gene so as to increase the expression amount of the p53 gene may be used.

An established cell line that can be stably subcultured is preferred in consideration of, for example, ease of an operation and reproducibility. As an established cell line that normally expresses p53 and can be stably subcultured, human-derived cells are used from the viewpoints that a relationship between DNA damage and p53 activation in humans is reproducible and the mutagenicity of a test substance on human cells can be accurately evaluated. Of those, human lymphoblast-derived TK6 cells are used. In particular, a combination of a recombinant vector using the above-mentioned pGL4-Luc2 plasmid or pGL4-Luc2P plasmid with TK6 cells is preferred because a time for contact with a test substance can be shortened to a great extent. The above-mentioned culture cells are available from American Type Culture Collection (ATCC). For example, the TK6 cells are represented by ATCC No. CRL-8015. It should be noted that in the case of using those cells, it is preferred that the expression of wild-type p53 in the cells be confirmed in advance by DNA sequencing or the like.

### Transformation method

In the preparation of the transformant, host cells may be transiently or stably transfected with the recombinant vector used in the present invention. The "stable transfection" can also be expressed as "insertion into the genome of mammalian cells" or "incorporation into the genome of mammalian cells . " It is preferred that the cells be stably transfected from the viewpoint that there is no need to consider a transformation efficiency. In addition, stable expression cells obtained by stably transfecting host cells with a vector are preferred from the viewpoints of work efficiency and the reproducibility of a mutagenicity test as well.

A method for transfection with a vector has already been well known. Specifically, for example, first, host cells may be plated in a dish having a diameter of from about 6 to 10 cm at from about 10⁵ to 10⁷ cells and cultured using, for example, an MEM medium containing serum at from about 5 to 10% under the conditions of 5% CO₂ and a saturated humidity at 37°C for from about several hours to overnight. The thus cultured cells may be transfected with the above-mentioned DNA. The purity of DNA with which the host cells are transfected is desirably almost comparable to that of plasmid DNA purified by a CsCl density gradient centrifugation method.

The amount of DNA for transfection is, for example, preferably from 0.1 to 1.0 ng, more preferably from 0.2 to 0.5 ng per 10⁵ cells when about 10⁵ cells are plated per dish (well).

As a method for transfection with DNA, a general method such as an electroporation method, a calcium phosphate method, or a lipofection method may be adopted without any limitation. Of those, an electroporation method is preferred from the viewpoints of simpleness of an operation and reproducibility. When transfection with a vector is performed by such method, ingeneral, the transformant is capable of transiently expressing a reporter gene. Accordingly, the transformant may be subjected to a mutagenicity test immediately after transfection with DNA.

In addition, cells stably expressing an expression vector may be produced by means known to a person skilled in the art. For example, cells stably transfected with a recombinant vector may be selected by the following method. After the transfection with the vector, host cells properly transfected with the recombinant vector may be selected by, for example, using as an indicator the activity of a cell selection marker gene for drug resistance or the like, the activation of a reporter gene with a positive control substance, or the fact that the presence of these genes can be detected. In this case, cells exhibiting luciferase activity two-fold or more as high as that at a basal level when the positive control substance is added are regarded as cells in which the reporter gene is activated.

As another means for preparing cells stably transfected with a recombinant vector, there is also given means using a virus vector that can incorporate a gene for transfection into the genome of mammalian cells, such as a retrovirus vector or a lentivirus vector. A specific method of carrying out the means is known to a person skilled in the art.

In order to obtain high luciferase activity, in the present invention, the amount of the recombinant vector for transfection is preferably from 0.1 to 1.0 (ng/well), more preferably from 0.2 to 0.5 (ng/well) in the case of, for example, using a 96-well plate.

The transformant used in the present invention may be suitably used as a cell to be subjected to a mutagenicity test to be described later.

A specific aspect of the transformant used in the present invention is exemplified by, but not limited to, the following aspect:
(a) a TK6 cell transiently harboring a recombinant vector including a p53 binding sequence, a minimal promoter that can function in a mammalian cell, and a luc2 gene linked so that the gene can be expressed by the minimal promoter;
(b) a TK6 cell stably harboring a recombinant vector including a p53 binding sequence, a minimal promoter that can function in a mammalian cell, and a luc2 gene linked so that the gene can be expressed by the minimal promoter;
(c) a TK6 cell transiently harboring a recombinant vector including a p53 binding sequence, a minimal promoter that can function in a mammalian cell, and a luc2P gene linked so that the gene can be expressed by the minimal promoter; or
(d) a TK6 cell stably harboring a recombinant vector including a p53 binding sequence, a minimal promoter that can function in a mammalian cell, and a luc2P gene linked so that the gene can be expressed by the minimal promoter.

### (III) Mutagenicity test method

A mutagenicity test method of the present invention is a method including the steps of:
(1) culturing the above-mentioned transformant in the presence of a test substance; and
(2) assessing whether or not the test substance affects the expression amount of a reporter gene in the transformant by comparing the expression amount of the reporter gene in the transformant brought into contact with the test substance to the expression amount of the reporter gene in the transformant not brought into contact with the test substance.

### Test substance

The kind of the test substance is not particularly limited. The test substance may be, in addition to a low-molecular-weight compound or a high-molecular-weight compound, for example, a mixture of a plurality of kinds of substances such as a food, a processed food, waste, or incinerated garbage.

### Step (1)

Hereinafter, a specific aspect of the step (1) is exemplified. In the present invention, a transformant is cultured in the presence of a test substance as described below. First, a transformant is plated in a cell culture vessel, and is preferably cultured for from about 8 hours to overnight (from about 0.5 to 1 hour in the case of using non-adherent cells such as TK6 cells) in advance. A culture temperature may be set to a temperature suited for cells of interest, and may be set to generally from about 36 to 38°C, preferably about 37°C. A culture medium is not particularly limited, and a culture medium suited for cells of interest may be used. The number of cells is not particularly limited, and about 1×10⁴ to 1×10⁵ cells are generally plated per well in the case of, for example, using a 96-well plate.

Next, a test substance is added to the cell culture medium. The test substance is added directly or after dissolved in a solvent. As the solvent, for example, distilled water, a buffer that does not affect cells, such as PBS (-), dimethyl sulfoxide (DMSO), ethanol, or methanol may be used. The test substance may be added so that the final concentration in the cell culture medium is generally from about 0.1 to 1 wt%. In the case of a processed food, its mixture with a buffer that does not affect cells, such as PBS (-), is added to the culture medium at 10 vol% or less, or the test substance extracted with an organic solvent such as methanol and concentrated may be added so that the final concentration is generally from about 0.1 to 1 wt%. When the test substance is added after dissolved in the solvent, the solvent is added to the culture medium of a control transformant in the same amount as the addition amount of a test substance solution.

After the test substance has been added to the cell culture medium, the culture is preferably continued in the same temperature range as described above for generally from about 4 to 10 hours, preferably from about 5 to 9 hours. In one aspect of the present invention, the culture may be performed for from about 6 to 10 hours, preferably from about 7 to 9 hours.

One preferred aspect of the step (1) is the step of:
(1) culturing the above-mentioned transformant in the presence of a test substance for from about 4 to 10 hours.

### Step (2)

After that, the expression amounts of the reporter gene are compared between the transformant brought into contact with the test substance and the transformant not brought into contact with the test substance. Hereinafter, a specific aspect of the step (2) is exemplified. In the comparison, the expression amounts of the reporter gene in both the cells may each be measured to compare both the measured values, or the expression amounts of the reporter gene in both the cells may also be directly qualitatively compared by visual observation or the like. Alternatively, the expression amounts of the reporter gene to be compared between the transformant brought into contact with the test substance and the transformant not brought into contact with the test substance may be relative values of the measured expression amounts of the reporter gene to the expression amount of another control gene (internal control). Such expression amounts of the reporter gene are also called the expression amounts of the reporter gene normalized or corrected with a control gene.

The expression amounts of the reporter gene may each be measured by a method depending on the kind of the reporter gene. For example, when the reporter gene is a firefly luciferase gene, the cells are each washed with PBS (-) or the like, a cell lysis agent containing a surfactant is then added to lyse the cells, luciferin, which is a luciferase substrate, is added to the cell lysate, and a light emission amount based on degradation of luciferin is quantified using a luminometer. Thus, luciferase activities can be measured.

The reporter assay may be performed by manual work, but may be quickly and simply performed by using the so-called high-throughput screening (The Tissue Culture Engineering, vol. 123, No. 13, 521-524, US 5, 670, 113 A) to be performed automatically using a machine.

When the expression amount of the reporter gene is a relative value to the expression amount of another control gene, the control gene is not particularly limited as long as the gene differs from the reporter gene. Specific examples of the control gene include: an endogenous gene in cells, such as a housekeeping gene (e.g., a GADPH gene or a β-actin gene); and a known exogenous reporter gene different from the above-mentioned reporter gene. When the reporter gene is used as the control gene, a reporter gene linked so that the gene can be expressed by a promoter that constitutively functions in mammalian cells, such as an SV40 promoter, a TK promoter, or a CMV promoter, is preferred.

From the viewpoint of simpleness of an operation, the control gene is preferably a gene whose expression amount can be measured by the same method as in the reporter gene used in the present invention. For example, when the reporter gene used in the present invention is a firefly-derived lucif erase gene or an engineered product thereof , a luciferase gene encoding luciferase derived from an organism other than firefly, in particular, a luciferase protein using a substance other than luciferin as a substrate may be used. An example of such luciferase gene is a Sea pansy (scientific name: *Renilla reniformis*) -derived luciferase gene. The Sea pansy luciferase gene also includes a base sequence having additions, deletions, or substitutions of about one or several nucleotides in a wild-type base sequence, the base sequence encoding a protein having luciferase activity. It should be noted that Sea pansy luciferase generally uses coelenterazine as a substrate.

When the control gene is an exogenous reporter gene, a transformant expressing the control gene used in the present invention may be produced in accordance with the production method for a transformant harboring the recombinant vector including the reporter gene used in the present invention. That is, in this case, the transformant used in the present invention is, for example, a transformant harboring the recombinant vector including the reporter gene used in the present invention and the recombinant vector including the control gene. When the transformant used in the present invention transiently harbors the recombinant vector including the reporter gene used in the present invention, it is preferred that the recombinant vector including the control gene be also transiently harbored, or when the transformant used in the present invention stably harbors the recombinant vector including the reporter gene used in the present invention, it is preferred that the recombinant vector including the control gene be also stably harbored.

In the case of using p53 binding sequences of a group of genes whose transcription is activated by p53, when the expression amount of the reporter gene per cell in the transformant brought into contact with the test substance significantly increases depending on the concentration of the test substance as compared to the expression amount of the reporter gene per cell in the transformant not brought into contact with the test substance, the test substance can be assessed to be a mutagenic substance or a carcinogenic substance. A criterion for whether or not the expression amount significantly increases may be appropriately set based on, for example, a significant test using a known carcinogenic substance. For example, when the expression amount of the reporter gene per cell in the transformant brought into contact with the test substance is 130% or more in the TK6 cells as compared to the expression amount of the reporter gene per cell in the transformant not brought into contact with the test substance, the test substance can be assessed to be a mutagenic substance or a carcinogenic substance.

In addition, in the case of using p53 binding sequences of a group of genes whose transcription is suppressed by p53, when the expression amount of the reporter gene per cell in the transformant brought into contact with the test substance significantly reduces depending on the concentration of the test substance as compared to the expression amount of the reporter gene per cell in the transformant not brought into contact with the test substance, the test substance can be assessed to be a mutagenic substance or a carcinogenic substance. A criterion for whether or not the expression amount significantly reduces may be appropriately set based on, for example, a significant test using a known carcinogenic substance. For example, when the expression amount of the reporter gene per cell in the transformant brought into contact with the test substance is 75% or less in the TK6 cells as compared to the expression amount of the reporter gene per cell in the transformant not brought into contact with the test substance, the test substance can be assessed to be a mutagenic substance or a carcinogenic substance.

In addition, in the method of the present invention, an anti-mutagenic substance or an anti-carcinogenic substance may be selected by bringing the transformant into contact with the test substance together with a known mutagenic substance. That is, in a comparison among the expression amount of the reporter gene in a control transformant, the expression amount of the reporter gene in a transformant brought into contact with only a mutagenic substance, and the expression amount of the reporter gene in a transformant brought into contact with the mutagenic substance and the test substance, when the expression amount of the reporter gene changed by contact with the mutagenic substance is reduced by further contact with the test substance, preferably does not significantly increase as compared to the expression amount of the reporter gene in the control transformant, the test substance can be assessed to be an anti-mutagenic substance or an anti-carcinogenic substance. An example of such substance is an antioxidant substance.

In the method of the present invention, an aspect using, as the expression amount of the reporter gene, a relative value to the expression amount of another control gene is one particularly preferred aspect of the present invention from the viewpoint of an improvement in accuracy of assessment.

Thus, a mutagenicity test on a test substance is carried out by the method of the present invention.

### (IV) Kit for mutagenicity test

A kit for a mutagenicity test used in the present invention is a kit for a mutagenicity test, including a TK6 cell harboring a recombinant vector including a p53 binding sequence, a minimal promoter that can function in a mammalian cell, and a luc2 gene linked so that the gene can be expressed by the minimal promoter. The mutagenicity test of the present invention can be simply carried out with the kit used in the present invention.

The structure of this kit is not particularly limited. For example, the kit may include an appropriate reagent, various containers, and the like. Specific examples thereof include: a medium for culturing cells, a reagent such as a reagent for measuring luciferase activity (e.g. , a reagent for lysing cells or a substrate for firefly luciferase), and a container for properly storing the medium or the reagent; and a tool such as a dish for performing cell culture.

In addition, the kit used in the present invention may include a protocol including descriptions about a procedure for carrying out the mutagenicity test of the present invention. The protocol preferably includes such a description that cells are brought into contact with a test substance and then the culture is continued for from about 4 to 10 hours, preferably from about 5 to 9 hours. Examples

Hereinafter, the present invention is described more specifically by way of Examples and Test Examples. However, the present invention is by no means limited to these examples.

### Example 1 (production of recombinant vector)

An oligonucleotide formed of a base sequence including three repeats of a p53 binding sequence of p53R2 gene intron to be expressed by DNA damage (5'-CTGACATGCCCAGGCATGTCTTGACATGCCCAGGCATGTCTTGACATGCCCAGGCATG TCTA-3': SEQ ID NO: 10), and an oligonucleotide formed of a base sequence complementary to this base sequence (5'-GATCTAGACATGCCTGGGCATGTCAAGACATGCCTGGGCATGTCAAGACATGCCTGGG CATGTCAGGTAC-3': SEQ ID NO: 11) were each synthesized with a DNA synthesizer, and the oligonucleotides were annealed to form double-stranded DNA.

A plasmid pGL4.10 including a firefly luciferase gene (manufactured by Promega) was digested with restriction enzymes KpnI and BglII and then subj ected to electrophoresis using lowmelting point agarose (NuSieve 3:1 Agarose; manufactured by BMA), and 4, 214-bp DNA of interest was collected from a gel at a band portion. About 30 ng of the DNA and 1 ng of the DNA including three repeats of the p53 binding sequence were mixed and ligated with T4 ligase (DNA Ligationkit; manufactured by TaKaRa). Afterthat, *Escherichia coli* JM109 competent cells (manufactured by TaKaRa) were transfected with the reaction liquid. From several colonies of *Escherichia coli* exhibiting ampicillin resistance, plasmid DNA harbored by each of the colonies was purified with a Plasmid Mini kit (manufactured by QIAGEN) and confirmed for its DNA sequence (ABI Prism BigDye Terminater v3.0; manufactured by Applied Biosystems) using an RVprimer3 primer (manufactured by Promega) and a reverse primer designed so as to be annealed downstream of the multicloning site of pGL4.10 (5'-CTTAATGTTTTTGGCATCTTCCA-3': SEQ ID NO: 12). A reporter plasmid confirmed to have the p53 binding sequence correctly inserted therein was named pGL4-p53R2x3-Luc2.

### Example 2 (production of recombinant vector; repertoire of promoter; preparation of reporter plasmid including SV40 minimal promoter)

A plasmid PGV-p53R2x3-Luc prepared by the method described in Example 1 of JP 4243716 B2 was digested with restriction enzymes BglII and HindIII and then subjected to electrophoresis using low melting point agarose (NuSieve 3:1 Agarose; manufactured by BMA), and 198-bp DNA of interest was collected from a gel at a band portion and named an SV40 minimal promoter

The reporter plasmid pGL4-p53R2x3-Luc2 prepared in Example 1 was digested with restriction enzymes BglII and HindIII and then subjected to electrophoresis using low melting point agarose, and 4, 257-bp DNA of interest was collected from a gel at a band portion. About 3 ng of the DNA and 10 ng of the DNA of the SV40 minimal promoter were mixed and ligated with T4 ligase. After that, *Escherichia coli* JM109 competent cells were transfected with the reaction liquid. From several colonies of *Escherichia coli* exhibiting ampicillin resistance, plasmid DNA harbored by each of the colonies was purified with a Plasmid Mini kit (manufactured by QIAGEN) and confirmed for its DNA sequence using an RVprimer3 primer and a reverse primer. A reporter plasmid confirmed to have the SV40 minimal promoter sequence correctly inserted therein was named pGL4-p53R2x3-SV40-Luc2.

### Example 3 (production of recombinant vector; repertoire of promoter; preparation of reporter plasmid including tk minimal promoter)

A plasmid PGV-p53R2x3-tk-Luc prepared by the method described in Example 3 of JP 4243716 B2 was digested with restriction enzymes BglII and HindIII and then subjected to electrophoresis using low melting point agarose (NuSieve 3:1 Agarose; manufactured by BMA), and 181-bp DNA of interest was collected from a gel at a band portion and named a tk minimal promoter

The reporter plasmid pGL4-p53R2x3-Luc2 prepared in Example 1 was digested with restriction enzymes BglII and HindIII and then subjected to electrophoresis using low melting point agarose, and 4, 257-bp DNA of interest was collected from a gel at a band portion. About 3 ng of the DNA and 10 ng of the DNA of the tk minimal promoter were mixed and ligated with T4 ligase. After that, *Escherichia coli* JM109 competent cells were transfected with the reaction liquid. From several colonies of *Escherichia coli* exhibiting ampicillin resistance, plasmid DNA harbored by each of the colonies was purified with a Plasmid Mini kit (manufactured by QIAGEN) and confirmed for its DNA sequence using an RVprimer3 primer and a reverse primer. A reporter plasmid confirmed to have the tk minimal promoter sequence correctly inserted therein was named pGL4-p53R2x3-tk-Luc2.

### Example 4 (production of recombinant vector; repertoire of promoter; preparation of reporter plasmid including TATA minimal promoter)

An oligonucleotide formed of a base sequence including a TATA minimal promoter (5'-GATCTCGACTATAAAGAGGGCAGGCTGTCCTCTAAGCGTCACCACGACTTCA-3': SEQ ID NO: 15), and an oligonucleotide formed of a base sequence complementary to this base sequence (5'-AGCTTGAAGTCGTGGTGACGCTTAGAGGACAGCCTGCCCTCTTTATAGTCGA-3': SEQ ID NO: 16) were each synthesized with a DNA synthesizer, and the oligonucleotides were annealed to form double-stranded DNA, which was named a TATA minimal promoter.

The reporter plasmid pGL4-p53R2x3-Luc2 prepared in Example 1 was digested with restriction enzymes BglII and HindIII and then subjected to electrophoresis using low melting point agarose, and 4, 257-bp DNA of interest was collected from a gel at a band portion. About 3 ng of the DNA and 10 ng of the DNA of the TATA minimal promoter were mixed and ligated with T4 ligase. After that, *Escherichia coli* JM109 competent cells were transfected with the reaction liquid. From several colonies of *Escherichia coli* exhibiting ampicillin resistance, plasmid DNA harbored by each of the colonies was purified with a Plasmid Mini kit (manufactured by QIAGEN) and confirmed for its DNA sequence using an RVprimer3 primer and a reverse primer. A reporter plasmid confirmed to have the TATA minimal promoter sequence correctly inserted therein was named pGL4-p53R2x3-TATA-Luc2.

### Example 5 (reactivity of pGL4-p53R2×3-SV40-Luc2)

Wild-type p53 of a human lymphoblast cell line TK6 cell was confirmed to be normal by DNA sequencing (ABI Prism BigDye Terminater v3.0; manufactured by Applied Biosystems) using primers for sequencing 5'-ACACGCTTCCCTGGATTG-3' (SEQ ID NO: 17) and 5'-CTGTCAGTGGGGAACAAGAAGTGGAGA-3' (SEQ ID NO: 18).

TK6 cells were transiently transfected with the plasmid pGL4-p53R2×3-SV40-Luc2 produced in Example 2 by an electroporation method. Next, the cells were plated in a 96-well plate for lucif erase light emission measurement and for culture (#3610 manufactured by Corning) using a medium containing fetal bovine serum (FBS) at a final concentration of 10% in an RPMI1640 medium (manufactured by Nissui Pharmaceutical) so that the cell density became from 70 to 80% confluent. It should be noted that the amount of pGL4-p53R2x3-SV40-Luc2 for transfection was adjusted to 0.25 ng/well.

This transformant was brought into contact with adriamycin, which was known to cleave DNA through DNA intercalation, to confirm whether or not a luciferase production amount increased. More specifically, the obtained transformant cells were divided into 7 groups, in 6 groups of which adriamycin dissolved in DMSO was added so as to achieve final concentrations of 0.003 µg/mL, 0.01 µg/mL, 0.03 µg/mL, 0.1 µg/mL, 0.2 µg/mL, and 0. 7 µg/mL, respectively (final DMSO concentration: 0.3% (v/v)). The same volume of DMSO as the addition amount of the adriamycin solution was added in the remaining 1 group, which was used as a negative control. After the addition of adriamycin or DMSO, those cells were cultured at 37°C for 4, 8, and 16 hours. Next, after the removal of the medium, a cell lysis solution (manufactured by Promega) was added at 25 µl/well, and the whole was left to stand at room temperature for 15 minutes. The plate was set in a luminometer LB96P with an enzyme substrate automatic injector (manufactured by Berthold), and a substrate solution (manufactured by Promega) was automatically dispensed at 30 µL/well to measure luciferase activities.

Next, the same operation as described above was performed to measure luciferase activities except that the plasmid PGV-p53R2x3-Luc prepared in Example 1 of JP 4243716 B2 was used in place of pGL4-p53R2x3-SV40-Luc2.

FIG. 3 shows the results. In the cells transfected with the reporter plasmid pGL4-p53R2x3-SV40-Luc2, an increase in luciferase activity due to an increase in concentration of adriamycin added to the cells was found. That is, such an increase in luciferase activity due to an increase in concentration of adriamycin as to be sufficient for performing a mutagenicity test was found at the time of contact with adriamycin for 4 hours. In addition, very high luciferase activity was measured at the time of contact with adriamycin for 8 hours. On the other hand, in the cells transfected with PGV-p53R2x3-Luc, very small luciferase activity was measured at the time of contact with adriamycin for 8 hours. It should be noted that in the cells transfected with PGV-p53R2x3-Luc, it took 16 hours after contact with adriamycin to measure luciferase activity sufficient for performing a mutagenicity test (data not shown). This fact reveals that higher luciferase activity can be confirmed in a shorter time in the cells transfected with pGL4-p53R2x3-SV40-Luc2 than in the cells transfected with PGV-p53R2x3-Luc.

### Example 6 (Comparison between different human-derived cells)

Wild-type p53 of a human lymphoblast cell line TK6 was confirmed to be normal in the same manner as described above. The TK6 cells were transiently transfected with the plasmid pGL4-p53R2x3-SV40-Luc2 produced in Example 2. Next, the cells were plated in a 96-well plate for luciferase light emission measurement and for culture (#3610 manufactured by Corning) using a medium containing fetal bovine serum (FBS) at a final concentration of 10% in an RPMI1640 medium (manufactured by Nissui Pharmaceutical) so that the cell density became from 70 to 80% confluent. It should be noted that the amount of pGL4-p53R2x3-SV40-Luc2 for transfection was adjusted to 0.25 ng/well.

To the cells transfected with the plasmid, adriamycin dissolved in DMSO was added so as to achieve final concentrations of 0.003 µg/mL, 0.01 µg/mL, 0.03 µg/mL, 0.1 µg/mL, 0.2 µg/mL, and 0.7 µg/mL, followed by culture for 8 hours. Next, luciferase activities were measured in accordance with the method of Example 5.

Further, regarding a human breast cancer cell line MCF-7, a human liver cancer cell line HepG2, a human lung cancer cell line A549, a human colon cancer cell line HCT116, a human embryonic kidney cell line HEK293, and a human skin-derived fibroblast cell line NB1RGB each having normal wild-type p53, the cells were plated in a 96-well plate for luciferase light emission measurement and for culture (#3610 manufactured by Corning) and cultured at 37°C overnight using a medium containing fetal bovine serum (FBS) at a final concentration of 10%. Next, the cells cultured so that the cell density became from 70 to 80% were each transiently transfected with the plasmid pGL4-p53R2x3-SV40-Luc2 produced in Example 2 at 0.25 ng/well.

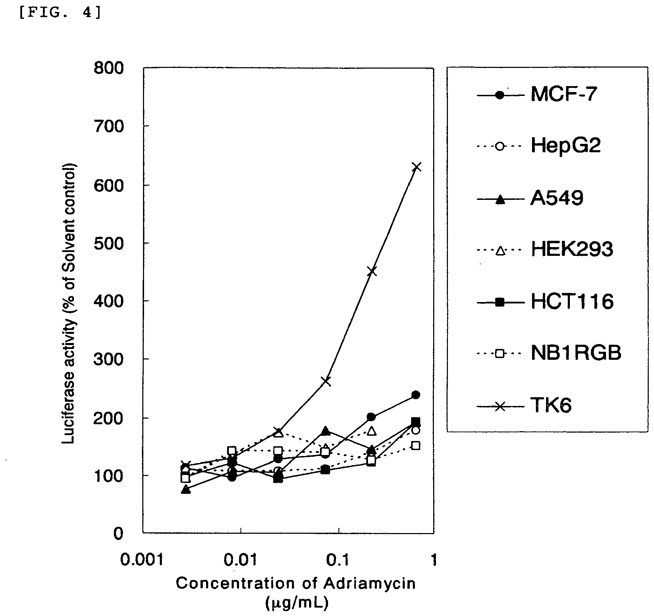

The obtained various transformants of the MCF-7 cells, the HepG2 cells, the A549 cells, the HCT116 cells, the HEK293 cells, and the NB1RGB cells were used to measure luciferase activities in the same manner as in the TK6 cells. FIG. 4 shows the results. In the TK6 cells transfected with pGL4-p53R2x3-SV40-Luc2, very high luciferase activity was found in the case of contact with adriamycin for 8 hours. On the other hand, in each of the MCF-7 cells, the HepG2 cells, the A549 cells, the HCT116 cells, the HEK293 cells, and the NB1RGB cells, only slight luciferase activity was found 8 hours after contact with adriamycin. This fact can confirm that an increase in luciferase activity in bringing the cells transfected with pGL4-p53R2×3-SV40-Luc2 into contact with adriamycin for 8 hours is remarkable in the TK6 cells.

### Example 7 (production of recombinant vector; repertoire of reporter gene; preparation of reporter plasmid including luc2P gene)

The same operations as in Examples 1 and 2 described above were performed except that pGL4.11 (manufactured by Promega) was used as a plasmid including a firefly luc if erase gene, and the obtained reporter plasmid was named pGL4-p53R2x3-SV40-Luc2P.

### Example 8 (Construction of stable expression cell of reporter gene)

TK6 cells were transiently transfected with the plasmid pGL4-p53R2x3-SV40-Luc2P produced in Example 7. Next, the cells were plated in a 96-well plate for culture (#3595 manufactured by Corning) at 1 cell/well using a medium containing fetal bovine serum (FBS) at a final concentration of 10% in an RPMI1640 medium (manufactured by Nissui Pharmaceutical). After that, the culture was continued for 1 month while the medium was appropriately exchanged, and the obtained cell colonies were divided into a 96-well plate for culture (#3595 manufactured by Corning) for subculture and a 96-well plate for luciferase light emission measurement and for culture (#3610 manufactured by Corning) for luciferase assay, and cultured for an additional 24 hours. After that, the cells divided for luciferase assay were measured for luciferase activities in accordance with the method of Example 5, and cells found to have luciferase activity were named TK6/p53R2x3-Luc2P.

### Example 9 (reactivity of TK6/p53R2x3-Luc2P)

The stable expression cells TK6/p53R2x3-Luc2P produced in Example 8 were plated in a 96-well plate for luciferase light emission measurement and for culture (#3610 manufactured by Corning) using a medium containing fetal bovine serum (FBS) at a final concentration of 10% in an RPMI1640 medium (manufactured by Nissui Pharmaceutical) so that the cell density became from 70 to 80% confluent.

Adriamycin dissolved in DMSO was added so as to achieve a final concentration of 0.2 µg/mL (final DMSO concentration: 0.3% (v/v)). The cells were cultured at 37°C for 2, 4, 6, and 8 hours. Next, after a lapse of each culture time, luciferase activity was measured in accordance with the method of Example 5.

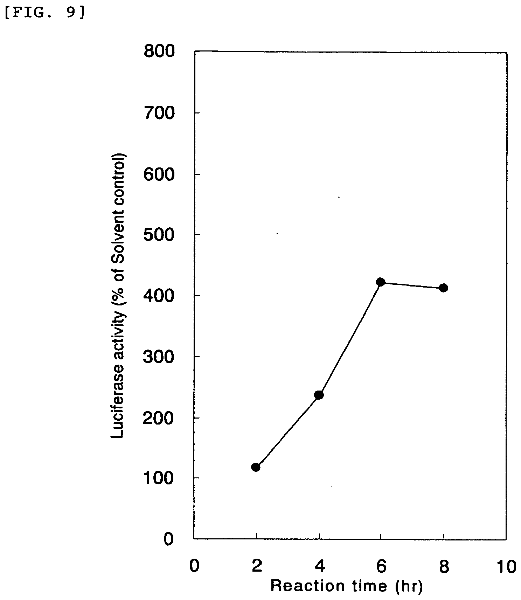

FIG. 9 shows the results. In the stable expression cells TK6/p53R2x3-Luc2P, an increase in luciferase activity due to an increase in concentration of adriamycin added to the cells was found. In addition, the maximum luciferase activity was achieved at the time of contact with adriamycin for 6 hours.

### Example 10 (reactivity of TK6/p53R2x3-Luc2P)

The stable expression cells TK6/p53R2x3-Luc2P produced in Example 8 were plated in a 96-well plate for luciferase light emission measurement and for culture (#3610 manufactured by Corning) so that the cell density became from 70 to 80% confluent in accordance with the method of Example 9.

The transformant was brought into contact with adriamycin to confirm whether or not a luciferase production amount increased. More specifically, the obtained transformant cells were divided into 7 groups, in 6 groups of which adriamycin dissolved in DMSO was added so as to achieve final concentrations of 0.003 µg/mL, 0.01 µg/mL, 0.03 µg/mL, 0.1 µg/mL, 0.2 µg/mL, and 0.7 µg/mL, respectively (final DMSO concentration: 0.3% (v/v)). The same volume of DMSO as the addition amount of the adriamycin solution was added in the remaining 1 group, which was used as a negative control. After the addition of adriamycin or DMSO, those cells were cultured at 37°C for 6 hours. Next, luciferase activities were measured in accordance with the method described in Example 5.

Next, the same operation as described above was performed to measure luciferase activities except that TK6 cells were transiently transfected with the plasmid pGL4-p53R2x3-SV40-Luc2 produced in Example 2 and cultured at 37°C for 8 hours after the addition of adriamycin or DMSO.

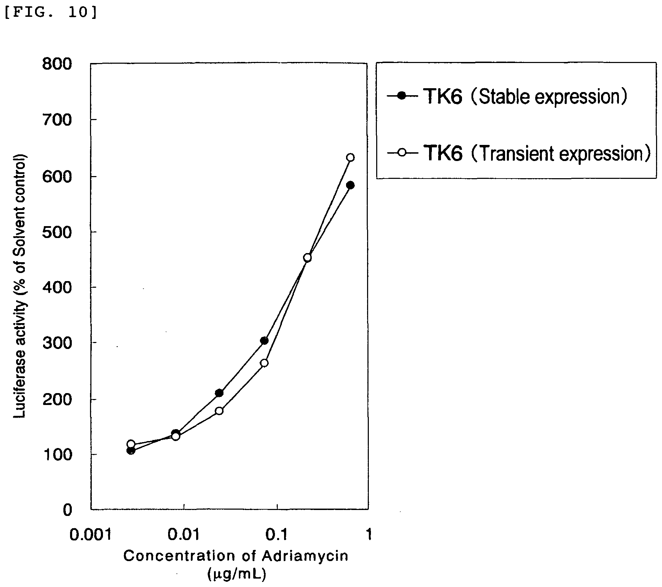

FIG. 10 shows the results. In the stable expression cells TK6/p53R2x3-Luc2P (TK6 (Stable expression)), an increase in luciferase activity due to an increase in concentration of adriamycin added to the cells was found. This fact and the results of Example 9 reveal that also in the case of using the stable expression cells TK6/p53R2x3-Luc2P, high luciferase activity can be confirmed in a short time as in the transient expression system (TK6 (Transient expression)) using the TK6 cells.

### Example 11 (construction of cells stably expressing internal control gene)

The cells TK6/p53R2x3-Luc2P produced in Example 8 were transiently transfected with a pRL-SV40-Rluc plasmid (plasmid in which Sea pansy luciferase (Rluc) was linked downstream of an SV40 promoter) in accordance with the method of Example 8. Next, in accordance with the method of Example 8, cells stably expressing Luc2P and Rluc were acquired and named TK6/p53R2x3-Luc2P/Rluc.

### Example 12 (correction with internal control)

The cells TK6/p53R2x3-Luc2P/Rluc produced in Example 11 were plated into a 96-well plate for luciferase light emission measurement and for culture in accordance with the method of Example 9.

Next, the above-mentioned two kinds of cells were brought into contact with 5-aza-2'-deoxycytidine (5-aza-dC). More specif ically, the obtained cells were divided into 7 groups, in 6 groups of which 5-aza-dC dissolved in DMSO was added so as to achieve final concentrations of 0.1 µg/mL, 0.2 µg/mL, 0.7 µg/mL, 2.2 µg/mL, 6.7 µg/mL, and 20 µg/mL, respectively (final DMSO concentration: 0.3% (v/v)). The same volume of DMSO as the addition amount of the 5-aza-dC solution was added in the remaining 1 group, which was used as a negative control. After the addition of 5-aza-dC or DMSO, those cells were cultured at 37°C for 6 hours.

After the removal of the medium, the expression amount of the Luc2P gene was determined as a relative value to the expression amount of the Rluc gene. Specifically, through the use of a luminometer LB96P with an enzyme substrate automatic injector (manufactured by Berthold), first, a substrate solution for firefly luciferase measurement (manufactured by Promega) was dispensed at 30 µL/well to measure the expression amount of the Luc2P gene. After that, a substrate solution for Sea pansy luciferase measurement (manufactured by Promega) was further dispensed at 30 µL/well to determine the expression amount of the Rluc gene.

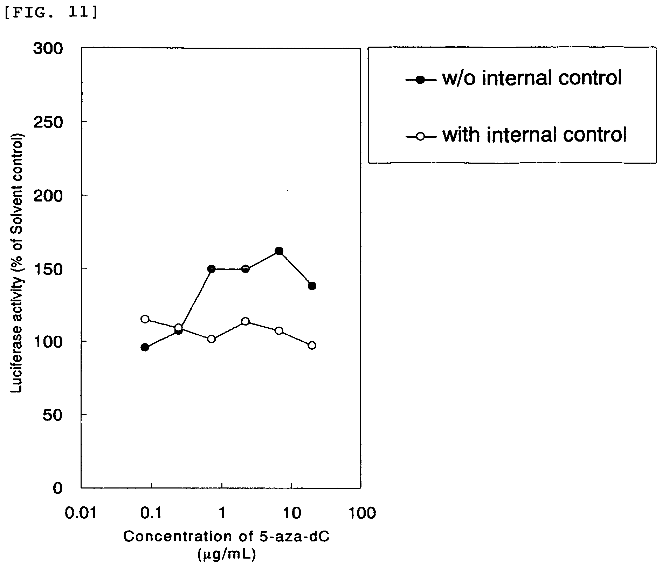

FIG. 11 shows the results. when the measured value for firefly luciferase (Luc2P) activity was not corrected with Sea pansy luciferase (Rluc) activity as an internal control (w/o internal control), an increase in measured value due to an increase in concentration of 5-aza-dC added to the cells was found. On the other hand, when the measured value for Luc2P activity was corrected with Rluc activity as an internal control (with internal control), no increase in measured value was found.

5-aza-dC is known to induce DNA demethylation through the inhibition of DNA methyltransferase activity to increase transcriptional activity per cell, but is considered not to have high mutagenicity. Accordingly, it is understood that the accuracy of the mutagenicity test of the present invention can be improved by correcting the Luc2P activity with the internal control.

### Reference Example 1

TK6 cells were transiently transfected with the plasmid PGV-p53R2x3-Luc prepared by the method described in Example 1 of JP 4243716 B2. Next, the cells were plated in a 96-well plate for luciferase light emission measurement and for culture (#3610 manufactured by Corning) using a medium containing fetal bovine serum (FBS) at a final concentration of 10% in an RPMI1640 medium (manufactured by Nissui Pharmaceutical) so that the cell density became from 70 to 80%.

To the cells transfected with the plasmid, adriamycin dissolved in DMSO was added so as to achieve final concentrations of 0.003 µg/mL, 0.01 µg/mL, 0.03 µg/mL, 0.1 µg/mL, 0.2 µg/mL, and 0.7 µg/mL, followed by culture for 8 hours. Next, luciferase activities were measured in accordance with the method of Example 5.

Similarly, a human breast cancer cell line MCF-7, a human liver cancer cell line HepG2, a human lung cancer cell line A549, a human colon cancer cell line HCT116, a human embryonic kidney cell line HEK293, and a human skin-derived fibroblast cell line NB1RGB each having normal wild-type p53 were each plated in a 96-well plate for luciferase light emission measurement and for culture (#3610 manufactured by Corning) and cultured at 37°C overnight using a medium containing fetal bovine serum (FBS) at a final concentration of 10%. Next, the culture cells in which the cell density became from 70 to 80% were transiently transfected with PGV-p53R2x3-Luc whose concentration was optimized so as to achieve the maximum reactivity in each of the culture cells.

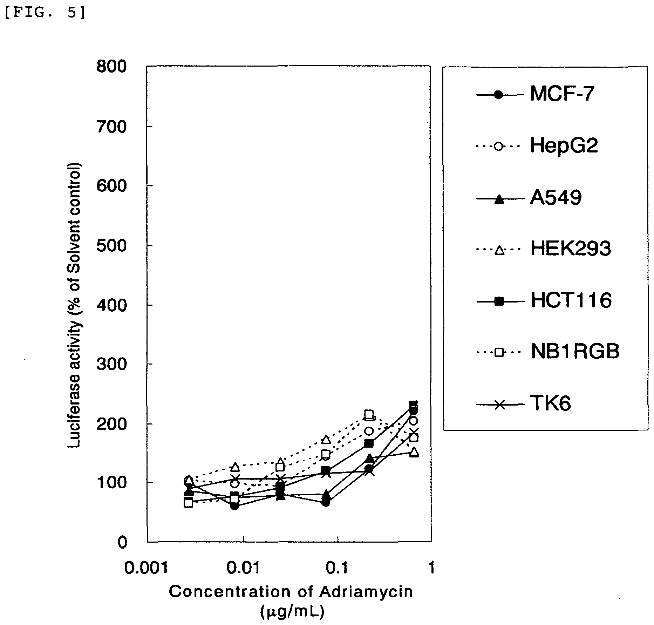

To the obtained various transformants of MCF-7, HepG2, A549, HCT116, HEK293, and NB1RGB, adriamycin dissolved in DMSO was added, followed by culture for 8 hours. After that, luciferase activities were measured in the same manner as in the TK6 cells. FIG. 5 shows the results.

As shown in FIG. 5, in each of the TK6 cells, the MCF-7 cells, the HepG2 cells, the A549 cells, the HCT116 cells, the HEK293 cells, and the NB1RGB cells transfected with PGV-p53R2x3-Luc, the contact with adriamycin for 8 hours resulted in only a slight increase in luciferase activity.

### Reference Example 2

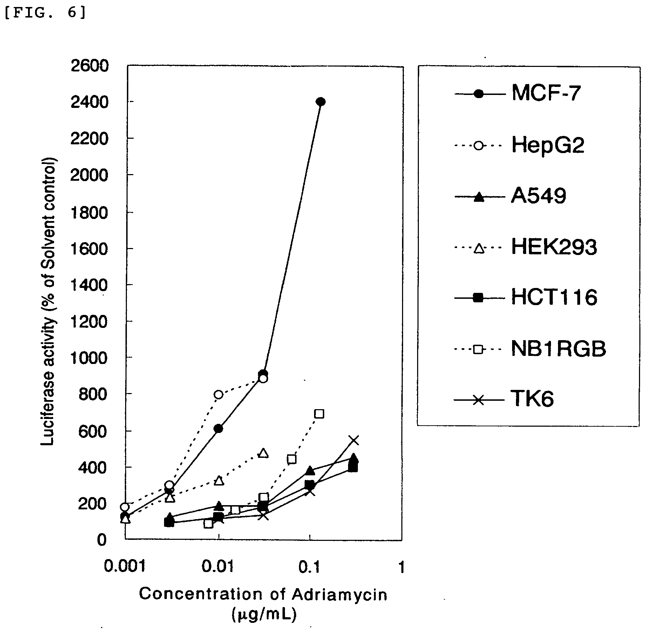

Transfection with plasmid PGV-p53R2x3-Luc, culture, and luciferase activity measurement were performed in the same manner as in Reference Example 1 described above except that the transformant was cultured for the following culture time: 16 hours in TK6 cells; 24 hours in MCF-7, HepG2, A549, HCT116, and HEK293; and 18 hours in NB1RGB. FIG. 6 shows the results.

As shown in FIG. 6, in each of the TK6 cells, the MCF-7 cells, the HepG2 cells, the A549 cells, the HCT116 cells, the HEK293 cells, and the NB1RGB cells transfected with PGV-p53R2x3-Luc, high luciferase activity was found in culture for 16 hours or more after contact with adriamycin. When the respective cells are compared, in the case of the transfection with PGV-p53R2x3-Luc, higher luciferase activities were found in the MCF-7 cells and the HepG2 cells than in the other cells. FIGS. 5 and 6 reveal that culture needs to be performed for as long a time as 16 hours or more after the contact with adriamycin in the system transfected with PGV-p53R2x3-Luc.

The technology relating to Patent Literature 1 developed by the inventors of the present invention has received the Technology Award from The Japanese Society of Toxicology. This award is an award to be granted to young researchers who are members of the Society and have made an excellent contribution such as the practical application of a novel development technology in the development of a toxicity evaluation technology, and the number of awardees is limited to 3 or less per year. Thus, the technology has received a high reputation in society. The present invention, which has been made by further improving the technology according to Patent Literature 1 to shorten a test time to a great extent, is very useful in the industry.

### Sequence Listing Free Text

- SEQ ID NO: 8: luc2 gene
- SEQ ID NO: 9: plasmid
- SEQ ID NO: 12: primer
- SEQ ID NO: 17: primer
- SEQ ID NO: 18: primer
- SEQ ID NO: 19: luc2P gene
- SEQ ID NO: 20: plasmid

## Claims

1. A mutagenicity test method, comprising the steps of:
(1) culturing a transformant of a mammalian cell harboring a recombinant vector comprising a p53 binding sequence, a minimal promoter that functions in the mammalian cell, and a reporter gene linked so that the gene is expressed by the minimal promoter, in a presence of a test substance for from 4 to 10 hours; and
(2) assessing whether or not the test substance affects an expression amount of the reporter gene in the transformant by comparing an expression amount of the reporter gene in the transformant cultured in the step (1) to an expression amount of the reporter gene in the transformant free of contact with the test substance,
wherein the reporter gene is luc2, and the transformant is a TK6 cell transfected with the recombinant vector.

2. The mutagenicity test method according to claim 1, wherein the minimal promoter comprises an SV40 promoter.

3. The mutagenicity test method according to claim 1, wherein the p53 binding sequence, the minimal promoter, and the reporter gene are disposed in the recombinant vector in an order of the p53 binding sequence, the minimal promoter, and the reporter gene from an upstream side of the recombinant vector.

4. The mutagenicity test method according to claim 1, wherein the p53 binding sequence comprises a base sequence set forth in SEQ ID NO: 1:
5'-WRRCWWGYYY-3': SEQ ID NO: 1
in SEQ ID NO: 1, R's each represent a nucleotide including a purine base, W's each represent a nucleotide including adenine or thymine, and Y's each represent a nucleotide including a pyrimidine base.

5. The mutagenicity test method according to claim 1, wherein the p53 binding sequence comprises a base sequence set forth in SEQ ID NO: 2:
5'-WRRCWWGYYY WRRCWWGYYY-3': SEQ ID NO: 2
in SEQ ID NO: 2, R's each represent a nucleotide including a purine base, W's each represent a nucleotide including adenine or thymine, and Y's each represent a nucleotide including a pyrimidine base.

6. The mutagenicity test method according to claim 1, wherein the expression amount of the reporter gene is a relative value to that of a control gene.

7. Use of a transformant for the mutagenicity test according to any one of claims 1 to 6, wherein the transformant is a TK6 cell harboring a recombinant vector comprising a p53 binding sequence, a minimal promoter that functions in a mammalian cell, and a reporter gene linked so that the gene is expressed by the minimal promoter, wherein the reporter gene is luc2.

8. Use of a kit for the mutagenicity test according to any one of claims 1 to 6, wherein the kit comprises the transformant according to claim 7.

## Patentansprüche

1. Mutagenitätstestverfahren, umfassend die Schritte:
(1) Kultivieren einer Transformante einer Säugerzelle, die einen rekombinanten Vektor beherbergt, umfassend eine p53-bindende Sequenz, einen Minimalpromotor, der in der Säugerzelle funktioniert und ein Reportergen, das so verbunden ist, daß das Gen durch den Minimalpromotor exprimiert wird, in Anwesenheit einer Testsubstanz für von 4 bis 10 Stunden und
(2) Bestimmen, ob die Testsubstanz eine Expressionsmenge des Reportergens in der Transformante beeinflußt oder nicht, durch Vergleichen einer Expressionsmenge des Reportergens in der in Schritt (1) kultivierten Transformante mit einer Expressionsmenge des Reportergens in der Transformante ohne Kontakt mit der Testsubstanz,
wobei das Reportergen luc2 ist und die Transformante eine TK6-Zelle ist, die mit dem rekombinanten Vektor transfiziert ist.

2. Mutagenitätstestverfahren nach Anspruch 1, wobei der Minimalpromotor einen SV40-Promotor umfaßt.

3. Mutagenitätstestverfahren nach Anspruch 1, wobei die p53-bindende Sequenz, der Minimalpromotor und das Reportergen in dem rekombinanten Vektor von einer *upstream* gelegenen Stelle des rekombinanten Vektors aus in Reihenfolge der p53-bindenden Sequenz, des Minimalpromotors und des Reportergens angeordnet sind.

4. Mutagenitätstestverfahren nach Anspruch 1, wobei die p53-bindende Sequenz eine Basensequenz wie in SEQ ID NO: 1 dargestellt umfaßt:
5'-WRRCWWGYYY-3': SEQ ID NO: 1
in SEQ ID NO: 1 stellt jedes R ein Nukleotid, enthaltend eine Purinbase dar, jedes W stellt ein Nukleotid, enthaltend Adenin oder Thymin dar und jedes Y stellt ein Nukleotid, enthaltend eine Pyrimidinbase dar.

5. Mutagenitätstestverfahren nach Anspruch 1, wobei die p53-bindende Sequenz eine Basensequenz wie in SEQ ID NO: 2 dargestellt umfaßt:
5'-WRRCWWGYYY WRRCWWGYYY-3': SEQ ID NO: 2
in SEQ ID NO: 2 stellt jedes R ein Nukleotid, enthaltend eine Purinbase dar, jedes W stellt ein Nukleotid, enthaltend Adenin oder Thymin dar und jedes Y stellt ein Nukleotid, enthaltend eine Pyrimidinbase dar.

6. Mutagenitätstestverfahren nach Anspruch 1, wobei die Expressionsmenge des Reportergens ein relativer Wert zu dem eines Kontrollgens ist.

7. Verwendung einer Transformante für den Mutagenitätstest nach einem der Ansprüche 1 bis 6, wobei die Transformante eine TK6-Zelle ist, die einen rekombinanten Vektor beherbergt, umfassend eine p53-bindende Sequenz, einen Minimalpromotor, der in einer Säugerzelle funktioniert und ein Reportergen, das so verbunden ist, daß das Gen durch den Minimalpromotor exprimiert wird, wobei das Reportergen luc2 ist.

8. Verwendung eines Kits für den Mutagenitätstest nach einem der Ansprüche 1 bis 6, wobei der Kit die Transformante nach Anspruch 7 umfaßt.

## Revendications

1. Procédé de test de mutagénicité, comprenant les étapes suivantes :
(1) la culture d'un transformant d'une cellule de mammifère abritant un vecteur recombinant comprenant une séquence de liaison de la p53, un promoteur minimal qui fonctionne dans la cellule de mammifère, et un gène rapporteur lié de telle façon que le gène est exprimé par le promoteur minimal, en présence d'une substance à tester de 4 à 10 heures ; et
(2) l'estimation si la substance à tester affecte ou pas une quantité d'expression du gène rapporteur dans le transformant par comparaison d'une quantité d'expression du gène rapporteur dans le transformant cultivé dans l'étape (1) à une quantité d'expression du gène rapporteur dans le transformant exempt de contact avec la substance à tester,
dans lequel le gène rapporteur est luc2, et le transformant est une cellule Tk6 transfectée avec le vecteur recombinant.

2. Procédé de test de mutagénicité selon la revendication 1, dans lequel le promoteur minimal comprend un promoteur du SV40.

3. Procédé de test de mutagénicité selon la revendication 1, dans lequel la séquence de liaison de la p53, le promoteur minimal, et le gène rapporteur sont disposés dans le vecteur recombinant dans un ordre de la séquence de liaison de la p53, le promoteur minimal, et le gène rapporteur à partir d'un côté en amont du vecteur recombinant.

4. Procédé de test de mutagénicité selon la revendication 1, dans lequel la séquence de liaison de la p53 comprend une séquence de bases représentée par SEQ ID NO : 1 :
5'-WRRCWWGYYY-3' : SEQ ID NO : 1
dans SEQ ID NO : 1, les R représentent chacun un nucléotide comprenant une base purique, les W représentent chacun un nucléotide comprenant de l'adénine ou de la thymine, et les Y représentent chacun un nucléotide comprenant une base pyrimidique.

5. Procédé de test de mutagénicité selon la revendication 1, dans lequel la séquence de liaison dé la p53 comprend une séquence de bases représentée par SEQ ID NO : 2 :
5'-WRRCWWGYYY WRRCWWGYYY-3' ; SEQ ID NO : 2
dans SEQ ID NO : 2, les R représentent chacun un nucléotide comprenant une base purique, les W représentent chacun un nucléotide comprenant de l'adénine ou de la thymine, et les Y représentent chacun un nucléotide comprenant une base pyrimidique.

6. Procédé de test de mutagénicité selon la revendication 1, dans lequel la quantité d'expression du gène rapporteur est une valeur relative par rapport à celle d'un gène témoin.

7. Utilisation d'un transformant pour le test de mutagénicité selon l'une quelconque des revendications 1 à 6, dans lequel le transformant est une cellule TK6 abritant un vecteur recombinant comprenant une séquence de liaison de la p53, un promoteur minimal qui fonctionne dans une cellule de mammifère, et un gène rapporteur lié de telle façon que le gène est exprimé par le promoteur minimal, dans lequel le gène rapporteur est luc2.

8. Utilisation d'un kit pour le test de mutagénicité selon l'une quelconque des revendications 1 à 6, où le kit comprend le transformant selon la revendication 7.
